# FASCICULE DE BREVET EUROPEEN

(11) **EP 1 541 120 B1**
(45) Date de publication et mention de la délivrance du brevet: **28.07.2010**
(21) Numéro de dépôt: 04292747.5
(22) Date de dépôt: 22.11.2004
(51) Int. Cl.: A61K 8/46, A61K 8/49, A61K 8/60, A61Q 5/00, A61Q 5/02, A61Q 5/06

(54) **Traitement de fibres kératiniques colorées avec une composition comprenant un tensioactif non ionique particulier et utilisation pour protéger la couleur**
Behandlung von gefärbten keratinischen Fasern mit einer Zusammensetzung enthaltend ein spezifisches, nicht-ionisches Tensid, und Verwendung um die Farbe zu schützen
Treatment of coloured keratinic fibers with a composition containing a specific, non-ionic surfactant, and use to protect the color

(30) Priorité: 11.12.2003 FR 0314549
(43) Date de publication de la demande: 15.06.2005
(73) Titulaire: L'Oréal, 75008 Paris (FR)
(72) Inventeur: Lalleman, Boris, 75015 Paris (FR); Kravtchenko, Sylvain, 92600 Asnieres (FR)
(74) Mandataire: Le Blainvaux Bellegarde, Françoise

(56) Documents cités:
- EP-A- 0 711 542
- US-A- 4 486 328
- US-A- 4 870 010
- US-A- 5 190 747
- US-A- 6 110 472
- US-A1- 2003 150 069
- US-B1- 6 432 146
- US-B2- 6 599 330
- K.SCHRADER: "Grundlagen und Rezepturen der Kosmetika" 1989, HÜTHIG BUCH VERLAG , HEIDELBERG , XP002286009 * alinéas [2.2.2.1], [2.2.3.2] *

## Description

La présente invention a pour objet un procédé de traitement de fibres kératiniques colorées ainsi que l'utilisation de tensioactifs non ioniques particuliers, ou leurs mélanges, pour protéger la coloration desdites fibres.

Plus précisément, le domaine de l'invention est celui du traitement des fibres kératiniques colorées, de préférence de fibres kératiniques humaines, comme notamment les cheveux.

Il existe essentiellement deux types de coloration des fibres kératiniques, la coloration dite permanente, et la coloration dite semi-permanente.

La première, aussi appelée coloration d'oxydation, consiste à mettre en oeuvre des précurseurs de colorants dits "d'oxydation", qui sont des composés incolores ou faiblement colorés. Une fois mélangés à des produits oxydants, au moment de l'emploi, ces précurseurs conduisent par un processus de condensation oxydative à des composés colorés et colorants. Dans ce cas, les colorations obtenues sont en général très tenaces et puissantes.

La seconde, connue aussi sous le nom de coloration directe, fait appel à des colorants directs, qui sont des composés colorants et colorés, non ioniques ou ioniques, capables d'apporter une modification plus ou moins marquée de la couleur naturelle des cheveux, résistant à plusieurs shampooings. Ces colorants peuvent être ou non mis en oeuvre en présence d'un agent oxydant.

Contrairement aux précurseurs de colorants d'oxydation, le colorant direct est une molécule relativement volumineuse qui ne pénètre pas facilement au coeur de la fibre. En conséquence, même si des progrès importants ont été réalisés dans ce domaine, le phénomène de dégorgement de la coloration lors des shampooings est toujours non négligeable, et cela même si le ou les colorants employés sont choisis parmi des espèces cationiques.

Par ailleurs, l'utilisation de certains colorants directs cationiques peut se traduire par une diminution des qualités d'usage des shampooings utilisés après coloration, notamment au niveau de la tenue de la mousse.

Le document US 6432146 décrit une composition de coloration comprenant un colorant d'oxydation et un colorant direct cationique. Les cheveux sont ensuite éventuellement lavés avec un shampooing standard. Aucune composition de shampooing n'est décrite dans ce document.

La présente invention a donc notamment pour objet de proposer un procédé de traitement des fibres kératiniques colorées permettant d'avoir un phénomène limité de dégorgement de la couleur lors des shampooings tout en bénéficiant de bonnes qualités d'usages pour lesdits shampooings.

Ainsi, la présente invention a pour objet un procédé de traitement de fibres kératiniques humaines dans lequel on met en oeuvre les étapes suivantes :
a) on réalise une étape de coloration desdites fibres au moyen d'une composition prête à l'emploi à base d'une composition colorante comportant au moins un colorant direct cationique possédant au moins un groupement hétérocyclique ;
b) éventuellement, on lave les fibres avec une composition lavante (A) comprenant au moins un tensioactif détergent anionique choisi parmi les alkyléthers sulfate et/ou les alkyl sulfate, on les rince et éventuellement on les sèche ou on les laisse sécher;
c) on lave les fibres avec une composition lavante (B) au moins un tensioactif non ionique choisi parmi les alkylpolyglucosides, les tensioactifs monoglycérolés ou polyglycérolés, et leurs mélanges, puis on rince les fibres et on les sèche ou on les laisse sécher.

Elle a de même pour objet un kit pour la mise en oeuvre dudit procédé, comprenant une composition colorante et éventuellement une composition comprenant au moins un agent oxydant ; éventuellement au moins une composition lavante (A), et au moins une composition lavante (B).

Elle a enfin pour objet l'utilisation d'une composition lavante comprenant à titre de tensioactif, au moins un tensioactif non ionique choisi parmi les alkylpolyglycosides, les tensioactifs monoglycérolés ou polyglycérolés, ou leurs mélanges, pour la limitation du dégorgement de la coloration et/ou la protection de la coloration des fibres kératiniques, en relation avec l'utilisation préalable d'une composition de coloration comprenant au moins un colorant direct cationique comportant au moins un groupement hétérocyclique.

On a en effet remarqué, de manière surprenante que l'emploi d'une composition lavante comprenant au moins un tensioactif non ionique particulier permettait de diminuer le phénomène de dégorgement de la couleur.

On a aussi constaté, par voie de conséquence, que le moindre dégorgement du ou des colorants se traduisait par un risque de tâchage de la peau et des tissus moins important.

De plus, la composition lavante selon l'invention présente de manière tout à fait avantageuse, des propriétés d'usage très satisfaisantes, comme une abondance de mousse présentant une bonne tenue dans le temps.

Enfin, la composition lavante permet une bonne protection de la coloration se traduisant par un moindre décapage de la coloration au fur et à mesure des shampooings.

Mais d'autres avantages et caractéristiques de l'invention apparaîtront plus clairement à la lecture de la description et des exemples qui vont suivre.

Ainsi que cela a été indiqué auparavant, le procédé selon l'invention consiste, dans une première étape, à réaliser une étape de coloration desdites fibres au moyen d'une composition prête à l'emploi à base d'une composition colorante comportant au moins un colorant direct cationique.

Par "colorant direct cationique", on entend au sens de l'invention un colorant porteur d'au moins un atome d'azote quaternisé.

Plus particulièrement, le ou les colorants directs cationiques sont choisis parmi les colorants xanthéniques, azoïques, azométhiniques et méthiniques.

On peut en particulier mettre en oeuvre un ou plusieurs colorants parmi ceux décrits dans la demande de brevet EP 1 025 834.

Par exemple, peuvent être utilisés de manière convenable, les composés suivants :

(I) G-N=N-J

dans laquelle le symbole G représente un groupement choisi parmi les structures G₁ à G₃ suivantes : 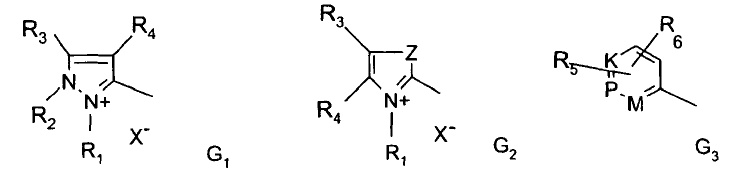 dans lesquelles,
R₁ désigne un radical alkyle en C₁-C₄. un radical phényle pouvant être substitué par un radical alkyle en C₁-C₄ ou un atome d'halogène choisi parmi le chlore, le brome, l'iode et le fluor ;
R₂ désigne un radical alkyle en C₁-C₄ ou un radical phényle;
R₃ et R₄, identiques ou différents, représentent un radical alkyle en C₁-C₄, un radical phényle, ou forment ensemble dans G₁ un cycle benzénique substitué par un ou plusieurs radicaux alkyle en C₁-C₄, alcoxy en C₁-C₄, ou NO₂, ou forment ensemble dans G₂ un cycle benzénique éventuellement substitué par un ou plusieurs radicaux alkyle en C₁-C₄ , alcoxy en C₁-C₄, ou NO₂ ;
R₃ peut désigner en outre un atome d'hydrogène ;
Z désigne un atome d'oxygène, de soufre ou un groupement -N⁺R₇(X⁻) ;
M représente un groupement -CH, -CR (R désignant alkyle en C₁-C₄), ou -N⁺R₈(X⁻)ᵣ ;
K représente un groupement -CH, -CR (R désignant alkyle en C₁-C₄),ou -N⁺R₈(X⁻)ᵣ ;
P représente un groupement -CH, -CR (R désignant alkyle en C₁-C₄),ou -N⁺R₈(X⁻)ᵣ ;
M, K ou P désignant un groupement -N⁺R₈(X⁻)ᵣ ;
r désigne zéro ou 1;
R₈ représente un atome O⁻, un radical alcoxy en C₁-C₄, ou un radical alkyle en C₁-C₄;
R₅ et R₆, identiques ou différents, représentent un atome d'hydrogène ou d'halogène choisi parmi le chlore, le brome, l'iode et le fluor, un radical alkyle en C₁-C₄, alcoxy en C₁-C₄, un radical -NO₂;
X⁻ représente un anion de préférence choisi parmi le chlorure, l'iodure, le méthyl sulfate, l'éthyl sulfate, l'acétate et le perchlorate;
le symbole J représente :
- (a) un groupement de structure J₁ suivante : 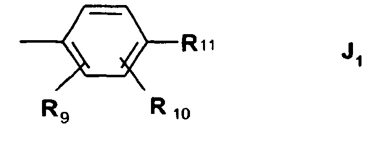 dans laquelle,
   R₉ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₁₂, -NR₁₃R₁₄, -NHCOalkyle en C₁-C₄, ou forme avec R₁₀ un cycle à 5 ou 6 chaînons contenant ou non un ou plusieurs hétéroatomes choisis parmi l'azote, l'oxygène ou le soufre;
   R₁₀ représente un atome d'hydrogène, un atome d'halogène choisi parmi le chlore, le brome, l'iode et le fluor, un radical alkyle en C₁-C₄, alcoxy en C₁-C₄ ou forme avec R₁₃ ou R₁₄ un cycle à 5 ou 6 chaînons contenant ou non un ou plusieurs hétéroatomes choisis parmi l'azote, l'oxygène ou le soufre;
   R₁₁ représente un atome d'hydrogène, un radical -OH, un radical -NHR₁₃, un radical -NR₁₄R₁₅;
   R₁₃ représente un atome d'hydrogène, un radical alkyle en C₁-C₄, un radical monohydroxyalkyle en C₁-C₄, polyhydroxyalkyle en C₂-C₄, un radical phényle;
   R₁₄ et R₁₅, identiques ou différents, représentent un radical alkyle en C₁-C₄ , un radical monohydroxyalkyle en C₁-C₄, polyhydroxyalkyle en C₂-C₄;
- (b) un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle,
   et notamment un groupement de structure J₂ suivante : 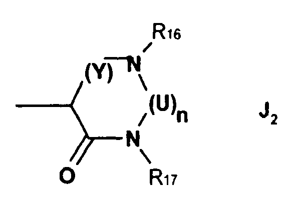 dans laquelle,
   R₁₆ et R₁₇, identiques ou différents, représentent un atome d'hydrogène , un radical alkyle en C₁₃-C₁₀, un radical phényle;
   Y désigne le radical -CO- ou le radical -C(CH₃)= ;
   n représente 0 ou 1, avec, lorsque n désigne 1, U désigne le radical -CO-.
   (II) : 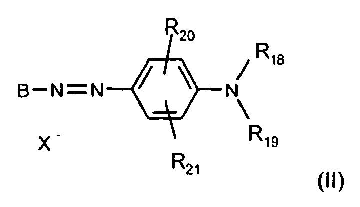 dans laquelle :
   R₁₈ représente un atome d'hydrogène ou un radical alkyle en C₁-C₄,
   R₁₉ représente un atome d'hydrogène, un radical alkyle pouvant être substitué par un radical -CN ou par un groupement amino, un radical 4'-aminophényle ou forme avec R₁₈ un hétérocycle éventuellement oxygéné et/ou azoté pouvant être substitué par un radical alkyle en C₁-C₄,
   R₂₀ et R₂₁, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
   X⁻ représente un anion de préférence choisi parmi le chlorure, le méthyl sulfate et l'acétate,
   B représente un groupement choisi par les structures B1 à B6 suivantes : 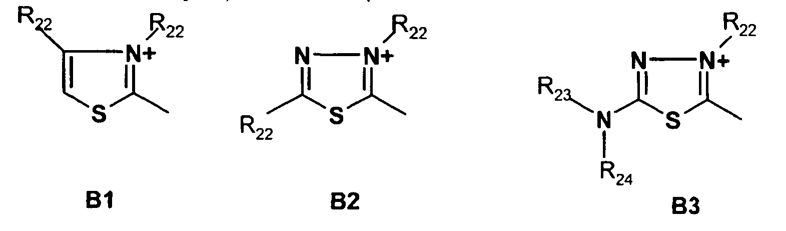 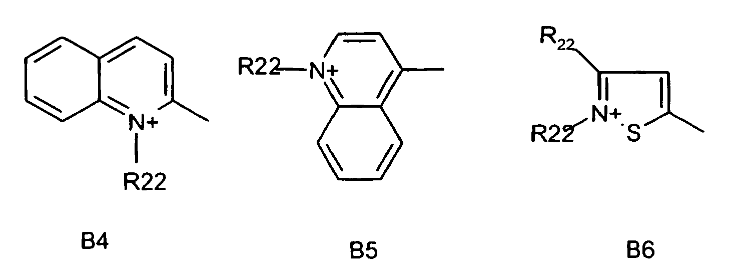 dans lesquelles R₂₂ représente un radical alkyle en C₁-C₄, R₂₃ et R₂₄, identiques ou différents, représentent un atome d'hydrogène ou un radical alkyle en C₁-C₄
   (III) et (IV) : 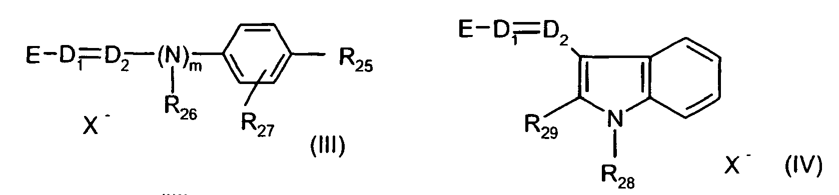 dans lesquel (III)
   R₂₅ représente un atome d'hydrogène, un radical alcoxy en C₁-C₄, un atome d'halogène tel que le brome, le chlore, l'iode ou le fluor ou un radical amino,
   R₂₆ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
   R₂₇ représente un atome d'hydrogène ou d'halogène tel que le brome, le chlore, l'iode ou le fluor,
   R₂₂ et R₂₃, identiques ou différents, représentent un atome d'hydrogène ou un radical alkyle en C₁-C₄,
   D₁ et D₂, identiques ou différents, représentent un atome d'azote ou le groupement -CH,
   m représente 0 ou 1,
   étant entendu que lorsque R₂₅ représente un groupement amino non substitué, alors D₁ et D₂ représentent simultanément un groupement -CH et m = 0,
   X- représente un anion de préférence choisi parmi le chlorure, le méthyl sulfate et l'acétate,
   E représente un groupement choisi par les structures E1 à E8 suivantes : 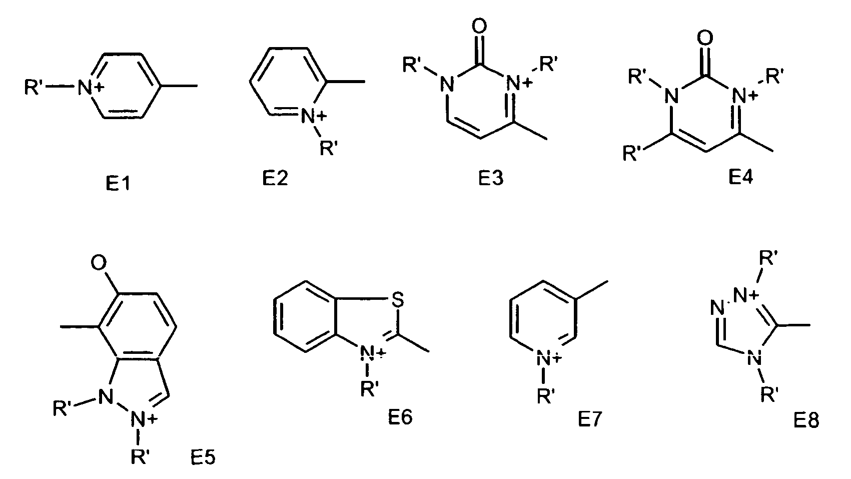 dans lesquelles R' représente un radical alkyle en C₁-C₄ ; lorsque m représente 0 et que D₁ représente un atome d'azote, alors E peut également désigner un groupement de structure E9 suivante : 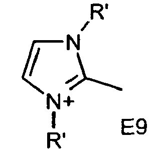 dans laquelle R' représente un radical alkyle en C₁-C₄.
   (V): 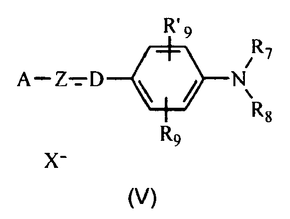 dans laquelle :
   Z et D représentent, identiques ou différents, un atome d'azote ou le groupement -CH,
   R₇ et R₈, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
   R₉ et R'₉, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyloxy,
   X⁻ représente un anion de préférence choisi parmi le chlorure, le méthyl sulfate et l'acétate,
   A représente un groupement choisi par les structures A1 à A18 suivantes : 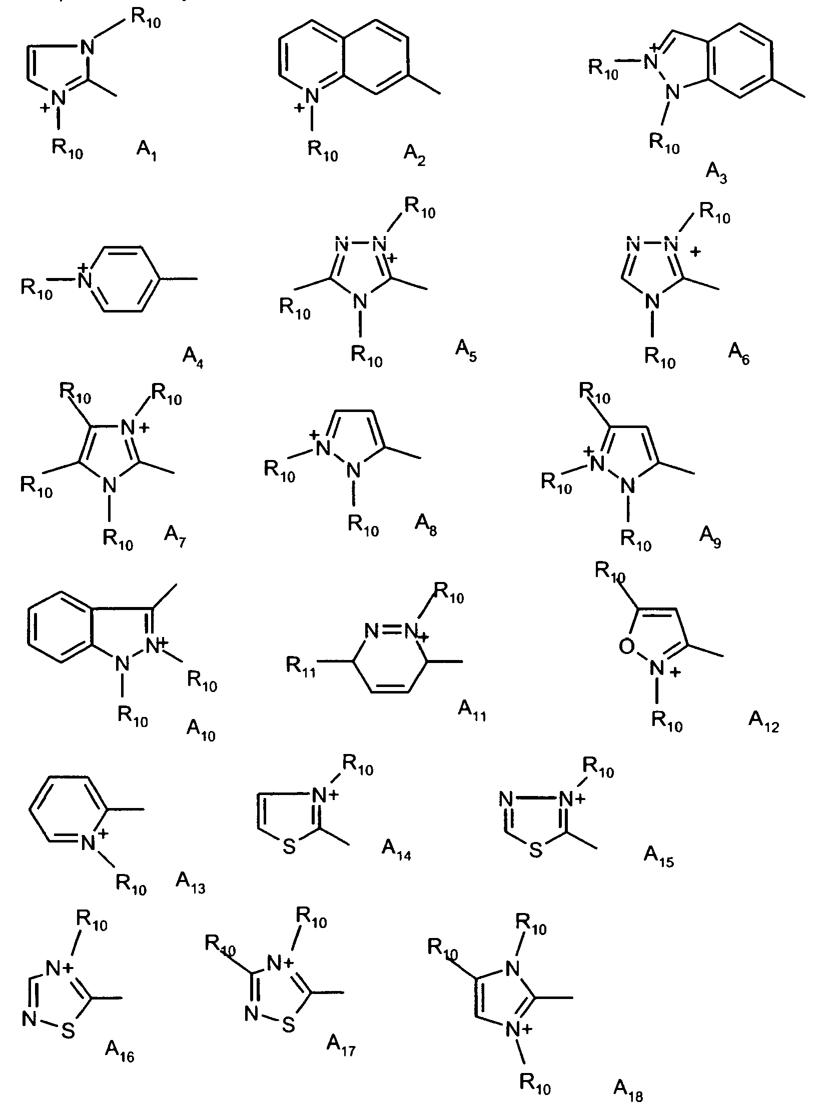 dans lesquelles R₁₀ représente un radical alkyle en C₁-C₄ pouvant être substitué par un radical hydroxyle et R₁₁ représente un radical alcoxy en C₁-C₄.

Conviennent de même les colorants cationiques décrits dans les demandes de brevet WO 95/01 772, WO 95/15 144, EP 714 954, EP 1 170 000 EP 1 166 753, EP 1 166 754 et EP 1 170 001, différents des colorants ci-dessus.

Selon l'invention, le colorant direct cationique est choisi en particulier parmi les colorants suivants :
- les colorants xanthéniques cationiques, parmi lesquels on utilise de préférence l'Acid Red 52,
- les colorants directs azoïques ou azométhiniques cationiques, parmi lesquels on peut utiliser de préférence le Basic Blue 41, le Basic Blue 67, le Basic Brown 1, le Basic Brown 4, le Basic Orange 31, le Basic Red 18, le Basic Red 22, le Basic Red 46, le Basic Red 51, le Basic Red 104, le Basic Violet 35, le Basic Yellow 45, le Basic Yellow 57 et le Basic Yellow 67, le Basic Yellow 87,
- les colorants directs méthiniques cationiques, comme notamment le Basic Red 14, le Basic Yellow 13 et le Basic Yellow 29.

De préférence, on utilise des colorants directs cationiques hétérocycliques, portant au moins une charge cationique sur un hétérocycle.

Tout particulièrement on utilise des colorants azoïques, méthiniques ou azométhiniques porteurs d'au moins une charge cationique sur un hétérocycle.

Le ou les colorants directs cationiques utilisés dans la composition colorante représentent avantageusement de 0,0001 à 20% en poids, plus particulièrement de 0,001 à 10% en poids, et de préférence de 0,01 à 5% en poids, par rapport au poids total de la composition colorante.

Il est à noter que la composition colorante peut éventuellement comprendre d'autres types de colorants directs comme par exemple des colorants directs non ioniques.
Si ce type de colorant est présent, sa teneur représente en général moins de 5 % en poids par rapport au poids de la composition colorante.

Conformément à un autre mode de réalisation de l'invention, la composition colorante comprend au moins une base d'oxydation et éventuellement au moins un coupleur.

Les composés de ce type utilisés classiquement dans le domaine de la coloration des fibres kératiniques, notamment humaines, peuvent être mis en oeuvre dans la composition colorante.

Ainsi, les bases d'oxydation peuvent notamment être choisies parmi les paraphénylènediamines, les bases doubles, les para-aminophénols, les ortho aminophénols et les bases d'oxydation hétérocycliques.

Parmi les paraphénylènediamines utilisables à titre de base d'oxydation dans les composition tinctoriales conformes à l'invention, on peut notamment citer les composés de formule (I) suivante et leurs sels d'addition avec un acide : 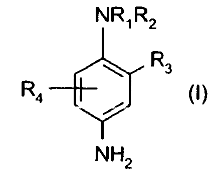 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₄ représente un atome d'hydrogène, d'halogène ou un radical alkyle en C₁-C₄.

Parmi les paraphénylènediamines de formule (I) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (II) suivante, et leurs sels d'addition avec un acide : 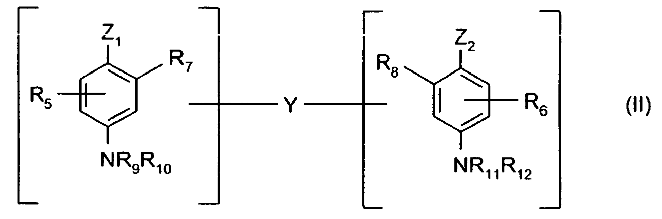 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀ R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (II) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols utilisables à titre de bases d'oxydation dans les compositions tinctoriales conformes à l'invention, on peut notamment citer les composés répondant à la formule (III) suivante, et leurs sels d'addition avec un acide : 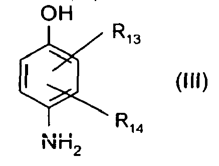 dans laquelle :
- R₁₃ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₄ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₃ ou R₁₄ représente un atome d'hydrogène.

A titre de para-aminophénols on peut aussi citer le 4 amino,6 [(5'-amino-2' hydroxy 3'methylphenyl)methyl] 2 méthylphenol et le bis (5 amino 2'hydroxyphenyl méthane et leurs sels d'addition avec un acide.

Parmi les para-aminophénols de formule (III) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, les dérivés pyrazolo-pyrimidiniques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-333 495 ou demandes de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1 -méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Parmi les dérivés pyrazolo-pyrimidiniques, on peut plus particulièrement citer les pyrazolo-[1,5-a]-pyrimidines de formule (IV) suivante, leurs sels d'addition avec un acide ou avec une base et leurs formes tautomères, lorsqu'il existe un équilibre tautomérique : 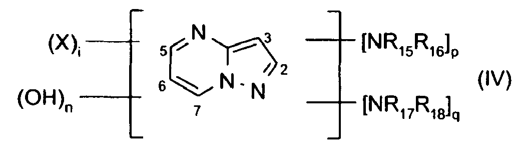 dans laquelle :
- R₁₅, R₁₆, R₁₇ et R₁₈, identiques ou différents désignent, un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical aminoalkyle en C₁-C₄ (l'amine pouvant être protégée par un radical acétyle, uréido ou sulfonyle), un radical (C₁-C₄)alkylamino alkyle en C₁-C₄, un radical di-[(C₁-C₄)alkyl] amino alkyle en C₁-C₄ (les radicaux dialkyles pouvant former un cycle carboné ou un hétérocycle à 5 ou 6 chaînons), un radical hydroxy(C₁-C₄)alkyl- ou di-[hydroxy(C₁-C₄) alkyl]-amino alkyle en C₁-C₄ ;
- les radicaux X désignent , identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle carboné ou un hétérocycle à 5 ou 6 chaînons), un radical hydroxy(C₁-C₄)alkyl ou di-[hydroxy(C₁-C₄ )alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;
- i vaut 0, 1, 2 ou 3 ; p vaut 0 ou 1 ; q vaut 0 ou 1 ; n vaut 0 ou 1 ; sous réserve que la somme p + q est différente de 0 ;
- lorsque p + q est égal à 2, alors n vaut 0 et les groupes NR₁₅R₁₆ et NR₁₇R₁₈ occupent les positions (2,3) ; (5,6) ; (6,7) ; (3,5) ou (3,7) ;
- lorsque p + q est égal à 1 alors n vaut 1 et le groupe NR₁₅R₁₆ (ou NR₁₇R₁₈) et le groupe OH occupent les positions (2,3) ; (5,6) ; (6,7) ; (3,5) ou (3,7) ;

Parmi les pyrazolo-[1 ,5-a]-pyrimidines de formule (IV) ci-dessus on peut notamment citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol ; le 2-(7-amino pyrazolo-[1,5-a]-pyrimidin-3-ylamino)-éthanol ; le 2-[(3-Amino-pyrazolo[1,5-a]pyrimidin-7-yl)-(2-hydroxy-éthyl)-amino]-éthanol ; le 2-[(7-Amino-pyrazolo[1,5-a]pyrimidin-3-yl)-(2-hydroxy-éthyl)-amino]-éthanol ; la 5,6-diméthyl pyrazolo-[1,5-a]-pyrimidine-3,7-diamine ; la 2,6-diméthyl pyrazolo-[1,5-a]-pyrimidine-3,7-diamine ; la 2, 5, N 7, N 7-tetraméthyl pyrazolo-[1,5-a]-pyrimidine-3,7-diamine ; et leurs sels d'addition et leurs formes tautomères, lorsqu'il existe un équilibre tautomérique.

Lorsque la composition colorante comprend une ou plusieurs bases d'oxydation, leur teneur représentent habituellement de 0,0005 à 12 % en poids, de préférence de 0,005 à 8 % en poids, par rapport au poids total de la composition colorante.

La composition colorante peut de plus comprendre au moins un coupleur associé à une ou plusieurs bases d'oxydation.
Ces coupleurs sont avantageusement choisis parmi ceux classiquement utilisés dans les compositions de teinture d'oxydation, c'est-à-dire des méta-phénylènediamines, des méta-aminophénols, des métadiphénols, des coupleurs hétérocycliques, et leurs sels d'addition avec un acide.

Ces coupleurs peuvent notamment être choisis parmi le 2-méthyl-5-amino-phénol, le 5-N-(β-hydroxyéthyl)-amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2,4-diamino-1-(β-hydroxyéthyloxy)-benzène, le 2-amino-4-(β-hydroxyéthylamino)-1-méthoxy-benzène, le 1,3-diamino-benzène, le 1,3-bis-(2,4-diaminophénoxy)-propane, le sésamol, l'α-naphtol, le 6-hydroxy-indole, le 4-hydroxy-indole, le 4-hydroxy-N-méthyl-indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, le 2,6-diméthyl- pyrazolo-[1,5-b]-1,2,4-triazole, le 2,6-diméthyl-[3,2-c]-1,2,4-triazole, le 6-méthyl-pyrazolo-[1,5-a]-benzimidazole, et leurs sels d'addition avec un acide.

Lorsqu'ils sont présents dans la composition colorante, la teneur en coupleur représente en général 0,0001 à 10 % en poids, et de préférence de 0,005 à 5 % en poids par rapport au poids de la composition colorante.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Il est à noter que la composition colorante mise en oeuvre lors de cette première étape peut comprendre tous les additifs usuels à ce type de composition. Ainsi, elle peut comprendre des additifs tels que des agents tensioactifs anioniques, cationiques, non ioniques, amphotères, zwittérioniques ou leurs mélanges ; des polymères ; des agents épaississsants ; des agents antioxydants ; des agents de pénétration ; des agents séquestrants ; des parfums ; des agents dispersants ; des agents filmogènes ; des filtres UV ; des vitamines ; des agents conservateurs ; des agents opacifiants, notamment.
Les adjuvants cités ci-dessus sont en général présents en quantité comprise, pour chacun d'eux, entre 0,01 et 20% en poids par rapport au poids de la composition.

La composition colorante comprend enfin un milieu approprié pour les fibres kératiniques qui est généralement constitué par de l'eau ou par un mélange d'eau et d'au moins un solvant organique acceptable sur le plan cosmétique, pour solubiliser les composés qui ne seraient pas suffisamment solubles dans l'eau.
A titre de solvant organique, on peut par exemple citer les monoalcools, linéaires ou ramifiés, de préférence saturés, comprenant 2 à 10 atomes de carbone, tels que l'alcool éthylique, l'alcool isopropylique ; les alcools aromatiques tels que l'alcool benzylique, l'alcool phényléthylique ; les polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol, l'hexylèneglycol (2-méthyl 2,4-pentanediol), le néopentylglycol et le 3-méthyl-1,5-pentanediol ; ainsi que les alkyléthers de diéthylèneglycol, notamment en C₁-C₄, comme par exemple, le monoéthyléther ou le monobutyléther du diéthylèneglycol, seuls ou en mélange.
Les solvants peuvent être présents dans des proportions de préférence comprises entre 1 et 40 % en poids environ par rapport au poids total de la composition tinctoriale, et encore plus préférentiellement entre 5 et 30 % en poids environ.

Habituellement, le pH des compositions colorantes varie entre 4 et 12, de préférence entre 6 et 11.

Selon un premier mode de réalisation, la composition prête à l'emploi appliquée sur les fibres kératiniques ne comprend pas d'agent oxydant. Dans ce cas, la composition prête à l'emploi correspond à la composition colorante.

Ce mode de réalisation est particulièrement approprié lorsque la composition ne comprend pas de base(s) d'oxydation ou de coupleur(s).

Selon un deuxième mode de réalisation, la composition prête à l'emploi est appliquée sur les fibres kératiniques, en présence d'un agent oxydant.

Ce mode de réalisation est approprié quelle que soit la nature des colorants présents dans la composition colorante.

Classiquement, l'agent oxydant est choisi notamment parmi le peroxyde d'hydrogène, le peroxyde d'urée, les bromates ou ferricyanures de métaux alcalins, les persels, les enzymes d'oxydoréduction telles que les laccases, les peroxydases et les oxydoréductases à 2 électrons, le cas échéant en présence de leur donneur respectif.

Dans le cas de ce deuxième mode de réalisation, la composition prête à l'emploi est obtenue par mélange, avant l'application sur les fibres, d'une composition colorante telle que décrite précédemment, avec une composition comprenant au moins un agent oxydant.

Selon ce procédé, on applique sur les fibres au moins une composition prête à l'emploi pendant un temps suffisant pour développer la coloration désirée, après quoi on rince éventuellement les fibres.

Le temps nécessaire au développement de la coloration est généralement compris entre 1 et 60 minutes et encore plus précisément 5 et 40 minutes.

La température à laquelle cette étape est réalisée varie en général entre 20°C et 80°C.

Une fois l'étape a) de coloration réalisée, éventuellement suivie d'un rinçage, on effectue éventuellement un lavage des fibres kératiniques au moyen d'une composition lavante (A) comprenant au moins un tensioactif détergent anionique choisi parmi les alkyléthers sulfate et/ou les alkyl sulfate, après quoi on rince lesdites fibres et éventuellement on les sèche ou on les laisse sécher.

Il est à noter que les parties alkyle des alkyléthers sulfate et/ou les alkyl sulfate listés ci-dessus comprennent avantageusement de 6 à 24 atomes de carbone, plus particulièrement de 8 à 18 atomes de carbone.

Habituellement, la teneur en tensioactif détergent anionique dans cette composition est comprise entre 4 à 50% en poids par rapport au poids de la composition lavante (A) et de préférence de 6 à 30% en poids par rapport au poids de la composition lavante (A).

La composition lavante (A) peut aussi comprendre un ou plusieurs tensioactifs non ioniques, amphotères ou zwittérioniques, anioniques doux.

Plus particulièrement, à titre de tensioactif non ionique, on peut citer :
- les alcools gras, oxyalkylénés ou polyglycérolés ;
- les alkylphénols dont la chaîne alkyle est en C₈-C₁₈, oxyalkylénés ;
- les amides gras oxyalkylénés ou polyglycérolés ;
- les amines grasses oxyalkylénées ;
- les huiles végétales oxyalkylénées ;
- les esters d'acides gras du sorbitan, éventuellement oxyalkylénés ;
- les esters d'acides gras du sucrose, éventuellement oxyalkylénés ;
- les esters d'acides gras du polyéthylèneglycol ;
- les alkylpolyglycosides ;
- les dérivés de N-alkyl glucamine ;
- les oxydes d'amines tels que les oxydes d'alkyl (C₁₀ - C₁₄) amines ou les oxydes de N-acylaminopropylmorpholine ;
- les copolymères d'oxyde d'éthylène et de propylène.

Par chaîne grasse, on entend une chaîne hydrocarbonée comprenant 6 à 30 atomes de carbone, de préférence de 8 à 24 atomes de carbone, linéaire ou ramifiée, saturée ou non.

Plus particulièrement, le nombre moyen de motifs oxyalkylénés est compris entre 2 et 30 motifs. De préférence, il s'agit de motifs oxyéthylénés, oxypropylénés ou leurs mélanges.

En ce qui concerne les tensioactifs polyglycérolés, ils comportent de préférence en moyenne 1 à 5 groupements glycérol et en particulier 1,5 à 4.

Pour ce qui a trait aux tensioactifs amphotères ou zwittérioniques, on peut mentionner sans avoir l'intention de s'y limiter, les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO⁻)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)₂-Y', avec z = 1 ou 2,
X' désigne le groupement -CH₂CH₂-COOH ou un atome d'hydrogène
Y' désigne -COOH ou le radical -CH₂-CHOH-SO₃H
R₂' désigne un radical alkyle d'un acide R₉-COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caproamphodiacetate, Disodium Capryloamphodiacetate, Disodium Cocoamphodipropionate, Disodium Lauroamphodipropionate, Disodium Caproamphodipropionate, Disodium Capryloamphodipropionate, Lauroamphodipropionic acid, Cocoamphodipropionic acid, disodium cocoamphocarboxy ethyl hydroxypropyl sulfonate. A titre d'exemple on peut citer le cocoamphodiacetate commercialisé sous la dénomination commerciale MIRANOL^{®} C2M concentré par la société Rhodia Chimie.

On peut aussi citer les dérivés de formule RCONH(CH₂)ₙN(Y₂-X₁)(Y₁-X₂), formule dans laquelle :
- Y₂ et Y₁ étant des radicaux divalents, identiques ou différents, choisis parmi les radicaux alkylènes linéaires ou ramifiés en C₁-C₆ éventuellement substitués par un ou plusieurs radicaux hydroxyles,
- X₁ et X₂ identiques ou différents hydrocarbonés en C₁-C₆ éventuellement interrompus par un ou plusieurs hétéroatomes et porteurs d'au moins une fonction sel d'acide choisie parmi les groupements carboxylate, sulfonate, sulfate, phosphate ou phosphonate,
- n désignant un nombre entier allant de 1 à 6

En ce qui concerne les tensioactifs anioniques doux, on peut citer notamment les composés de formules suivantes, ainsi que leurs mélanges :
- les acides alkyl éther carboxyliques polyoxyalkylénés,
- les acides alkylaryl éther carboxyliques polyoxyalkylénés,
- les acides alkylamido éther carboxyliques polyoxyalkylénés en particulier ceux comportant 2 à 50 groupements oxyde d'éthylène,
- les acides d'alkyl D galactoside uroniques
- les acylsarcosinates, les acylglutamates ;
- les esters d'alkylpolyglycosides carboxyliques ;
- les sels d'acides gras.

Tout particulièrement, on utilise des acides alkyl éther carboxyliques polyoxyalkylénés comme par exemple l'acide lauryl éther carboxylique (4,5 OE) commercialisé par exemple sous la dénomination AKYPO RLM 45 CA de KAO.

Lorsqu'ils sont présents, la teneur en tensioactifs anioniques doux, amphotères et/ou zwittérionique représente de 4 à 50 % en poids par rapport au poids total des tensioactifs présents dans la première composition lavante (A).

La composition lavante (A) peut de plus comprendre les additifs classiques dans le domaine comme par exemple ceux choisis parmi la liste non exhaustive tels que les agents réducteurs, les agents oxydants, les séquestrants, les adoucissants, les agents anti-mousse, les agents hydratants, les agents émollients, les agents alcalinisants, les plastifiants, les filtres solaires, les colorants directs ou d'oxydation, les pigments, les charges minérales, les argiles, les minéraux colloïdaux, les nacres, les agents nacrant, les parfums, les peptisants, les conservateurs, les polymères fixant ou non, les protéines, les vitamines, les agents antipelliculaires, les alcools aliphatiques ou aromatiques, et plus particulièrement l'éthanol, l'alcool benzylique, les polyols modifiés ou non tels que le glycérol, le glycol, le propylène glycol, le dipropylène glycol, le butylène glycol, le butyle diglycol, les silicones volatiles, les huiles minérales, organiques ou végétales, les cires oxyéthylénées ou non, les paraffines, les acides gras, les polymères épaississants associatifs ou non, les amides grasses, les esters gras, les alcools gras, etc.
Les adjuvants cités ci-dessus sont en général présents en quantité comprise, pour chacun d'eux, entre 0,01 et 20% en poids par rapport au poids de la composition.

Il est à noter que si la composition comprend un ou agents épaississants, leur teneur est comprise entre 0,01 et 20% en poids par rapport au poids de la composition lavante, de préférence de 0,01 à 3% en poids par rapport au poids de la composition lavante.

La composition selon l'invention peut contenir un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non et leurs mélanges.

Les compositions peuvent se présenter sous différentes formes galéniques tels qu'une lotion, un spray, une mousse aérosol, une pompe mousse, etc.

De préférence, la composition lavante (A) comprend au moins un agent conditionneur, de préférence cationique

Selon ce mode de réalisation, la teneur en agent conditionneur dans la composition lavante (A) est comprise entre 0.01 et 20% en poids par rapport au poids de la composition lavante (A).

Selon un mode de réalisation préféré, l'agent conditionneur est un polymère cationique et/ou ou une silicone volatile ou non de préférence une silicone aminée.

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP 337 354 et dans les demandes de brevets français FR 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3. 10⁶ environ.

Parmi les polymères cationiques, on peut citer plus particulièrement les polymères du type polyamine, polyaminoamide et polyammonium quaternaire.

Les polymères du type polyamine, polyaminoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de for- mules suivantes : 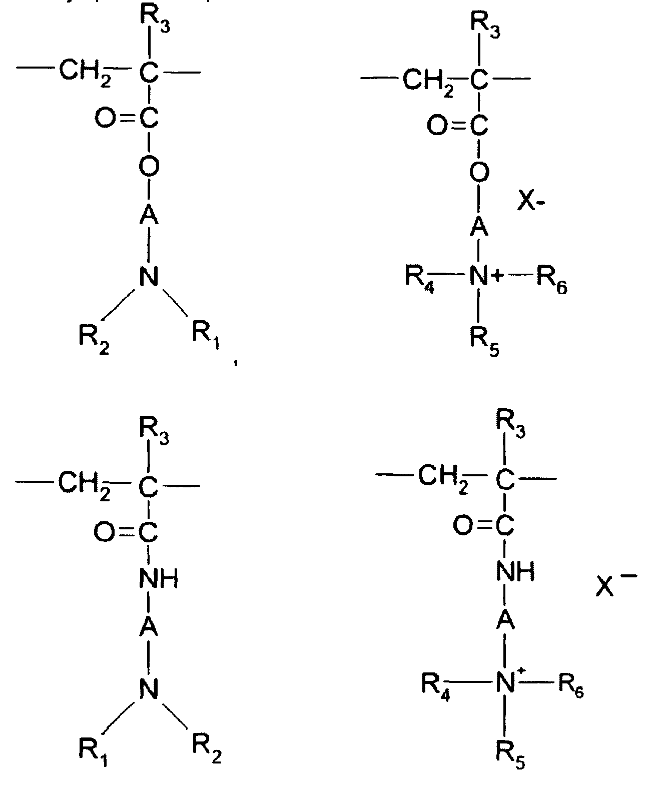 dans lesquelles:
   R₃, identiques ou différents, désignent un atome d'hydrogène ou un radical CH₃ ;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone; R 1 et R 2 , identiques ou différents, représentent hydrogène ou un groupe alkyle ayant de 1 à 6 atomes de carbone et de préférence méthyle ou éthyle ; X désigne un anion dérivé d'un acide minéral ou organique tel que un anion méthosulfate ou un halogénure tel que chlorure ou bromure.

   Les copolymères de la famille (1) peuvent contenir en outre un ou plusieurs motifs dérivant de comonomères pouvant être choisis dans la famille des acrylamides, méthacrylamides, diacétones acrylamides, acrylamides et mé-thacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de diméthyle tels que celui vendu sous la dénomination Hercofloc® par la société Hercules,
   - les copolymères d'acrylamide et de chlorure de méthacryloyloxyéthyltriméthylammonium décrit par exemple dans la demande de brevet EP-A-080976 et vendus sous la dénomination BINA QUAT P 100® par la société Ciba Geigy,
   - le copolymère d'acrylamide et de méthosulfate de méthacryloyloxyéthyltriméthylammonium vendu sous la dénomination Reten® par la société Hercules,
   - les copolymères vinylpyrrolidone / acrylate ou méthacrylate de dialkylami-noalkyle quaternisés ou non, tels que les produits vendus sous la dénomina- tion "Gafquat" par la société ISP comme par exemple "Gafquat 734" ou "Gafquat 755" ou bien les produits dénommés "Copolymer 845, 958 et 937". Ces polymères sont décrits en détail dans les brevets FR 2077143 et FR 2393573,
   - les terpolymères méthacrylate de diméthyl amino éthyle/ vinylcaprolactame/ vinylpyrrolidone tel que le produit vendu sous la dénomination Gaffix® VC 713 par la société ISP,
   - les copolymère vinylpyrrolidone / méthacrylamidopropyl dimethylamine commercialisés notamment sous la dénomination Styleze® CC 10 par ISP, et
   - les copolymères vinylpyrrolidone / méthacrylamide de diméthylaminopropyle quaternisé tel que le produit vendu sous la dénomination "Gafquat® HS 100" par la société ISP.
(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl- celluloses greffées notamment avec un sel de méthacryloyléthyl triméthylammonium, de méthacrylamidopropyl triméthylammonium, de diméthyl-diallylammonium.
   Les produits commercialisés répondant à cette définition sont plus particulièrement les produits vendus sous la dénomination "CELQUAT ® L 200" et "CELQUAT ® H 100" par la Société National Starch.
(4) Les polysaccharides cationiques décrits plus particulièrement dans les brevets US 3 589 578 et 4 031 307 tels que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR ® C13 S, JAGUAR ® C 15, JAGUAR ® C 17 ou JAGUAR ® C162 par la société RHODIA CHIMIE.
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets FR 2162025 et FR 2280361.
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohy-drine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets FR 2252840 et FR 2368508.
(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet FR 1583363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyami- noamide compris entre 0,5 : 1 et 1,8 : 1. De tels polymères sont notamment décrits dans les brevets US 3227615 et US 2961347.
   Des polymères de ce type sont en particulier commercialisés sous la dénomination "Hercosett 57" par la société Hercules Inc. ou bien sous la dé- nomination de "PD 170" ou "Delsette 101" par la société Hercules dans le cas du copolymère d'acide adipique/époxypropyl/diéthylène-triamine.
(9) les cyclopolymères d'alkyl diallyl amine ou de dialkyl diallyl ammonium tels que les homopolymères ou copolymères comportant comme constituant principal de la chaîne des motifs répondant aux formules (Va) ou (Vb) : 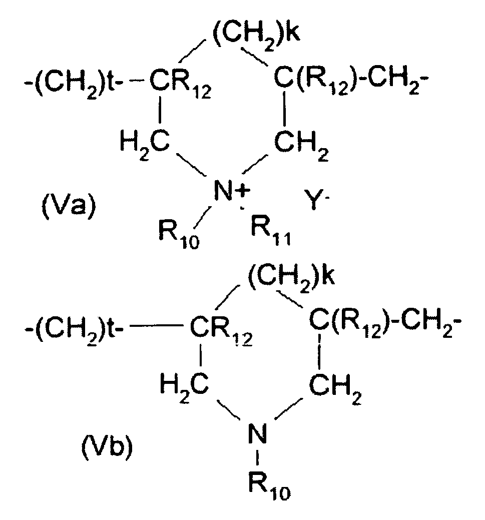 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁,peuvent désigner conjointement avec l'atome d'azote auquel ils sont rattachés, des groupements hétérocycliques, tels que pipéridinyle ou morpholinyle ; Y - est un anion tel que bromure, chlorure, acétate, borate, citrate, tartrate, bisulfate, bisulfite, sulfate, phosphate. Ces polymères sont notamment décrits dans le brevet FR 2080759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "Merquat® 100" par la société Nalco (et ses homologues de faibles masses moléculaires moyenne en poids) et les copolymères de chlorure de diallyldiméthylammonium et d'acrylamide commercialisés sous la dénomination "Merquat® 550".
(10) le polymère de diammonium quaternaire contenant des motifs récurrents répondant à la formule (VI) : 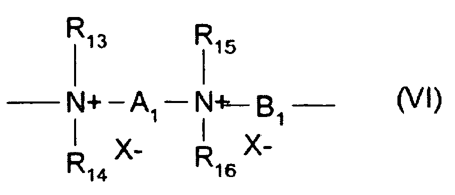 formule (VI) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R ₁₃ , R ₁₄ , R ₁₅ et R ₁₆ , ensemble ou
   séparément, constituent avec ies atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C₁-C₆ linéaire ou ramifié substitué par un groupement nitrile, ester, acyle, amide ou -CO-O-R₁₇ -D ou -CO-NH-R₁₇ - D où R₁₇ est un alkylène et D un groupement ammonium quaternaire ;
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique, de préférence Cl⁻, Br⁻;
   A₁, R₁₃ et R₁₅ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B₁ peut également désigner un groupement (CH₂)ₙ-CO-D-OC-(CH₂)ₙ-
   dans lequel D désigne : a) un reste de glycol de formule : -O-Z-O-, où Z désigne un radical hydrocarboné linéaire ou ramifié ou un groupement répondant à l'une des formules suivantes : -(CH₂-CH₂-O)x-CH₂-CH₂- / -[CH₂-CH(CH₃)-O]_{y}-CH₂-CH(CH₃)- où x et y désignent un nombre entier de 1 à 4, représentant un degré de polymérisation défini et unique ou un nombre quelconque de 1 à 4 représentant un degré de polymérisation moyen ; b) un reste de diamine bis-secondaire tel qu'un dérivé de pipérazine ; c) un reste de diamine bis-primaire de formule : -NH-Y-NH-, où Y désigne un radical hydrocarboné linéaire ou ramifié, ou bien le radical bivalent -CH₂-CH₂-S-S-CH₂-CH₂- ; d) un groupement uréylène de formule : -NH-CO-NH- ; n varie de 1 à 6.
   Ces polymères ont une masse moléculaire moyenne en nombre généralement comprise entre 1000 et 100000.
   Des polymères de ce type sont notamment décrits dans les brevets FR 2320330, FR 2270846, FR 2316271, FR 2336434 et FR 2413907 et les brevets US 2273780, US 2375853, US 2388614, US 2454547, US 3206462, US 2261002, US 2271378, US 3874870, US 4001432, US 3929990, US 3966904, US 4005193, US 4025617, US 4025627, US 4025653, US 4026945 et US 4027020.
   On peut utiliser plus particulièrement les polymères qui sont constitués de motifs récurrents répondant à la formule : 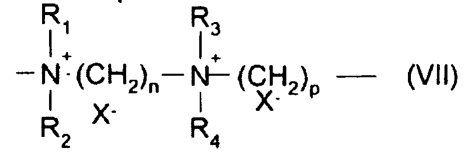 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X - est un anion dérivé d'un acide minéral ou organique.
   Un composé de formule (VII) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).
(11) les polymères de polyammonium quaternaires constitués de motifs de formule (VIII): 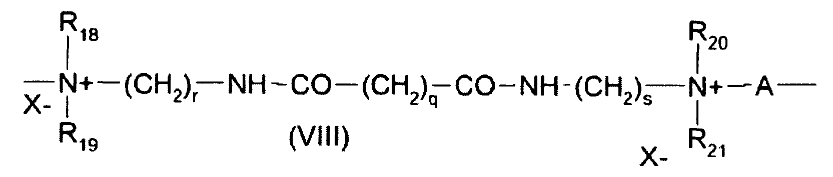 formule dans laquelle :
   R₁₈, R₁₉, R₂₀ et R₂₁ , identiques ou différents, représentent un atome d'hydrogène ou un radical méthyle, éthyle, propyle, ®-hydroxyéthyle, ®-hydroxypropyle ou -CH₂CH₂(OCH₂CH₂)ₚOH, où p est égal à 0 ou à un nombre entier compris entre 1 et 6, sous réserve que R₁₈, R₁₉, R₂₀ et R₂₁ ne représentent pas simultanément un atome d'hydrogène, r et s, identiques ou différents, sont des nombres entiers compris entre 1 et 6, q est égal à 0 ou à un nombre entier compris entre 1 et 34, X⁻ désigne un anion tel qu'un halogénure, A désigne un radical d'un dihalogénure ou représente de préférence -CH₂-CH₂-O-CH₂-CH₂-.

   De tels composés sont notamment décrits dans la demande de brevet EP 122 324.
   On peut par exemple citer parmi ceux-ci, les produits "Mirapol® A 15", "Mirapol® AD1", "Mirapol® AZ1" et "Mirapol® 175" vendus par la société Miranol.
(12) Les polymères quaternaires de vinylpyrrolidone et de vinylimidazole tels que par exemple les produits commercialisés sous les dénominations Luvi-quat® FC 905, FC 550 et FC 370 par la société BASF.
(13) Les polyamines comme le Polyquart® H vendu par COGNIS, référencé sous le nom de « POLYETHYLENEGLYCOL (15) TALLOW POLYAMINE » dans le dictionnaire CTFA.
(14) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylmé-thacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl tri-méthylammonium (20/80 en poids) sous forme de dispersion contenant 50 % en poids dudit copolymère dans de l'huile minérale. Cette dispersion est commercialisée sous le nom de Salcare ® SC 92 par la société Ciba Geigy. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de Salcare ® SC 95 et Salcare ® SC 96 par la société Ciba Geigy.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose quaternaires tels que les produits vendus sous la dénomination JR 400 par la société Amerchol, les cyclopolymères cationiques, en particulier les homopolymères ou copolymères de chlorure de diméthyldiallylammonium, vendus sous les dénominations Merquat® 100, Merquat® 550 et Merquat® S par la société Nalco, les polysaccharides cationiques tels que les gommes de guar modifiées par un sel de 2,3-époxypropyl triméthylammonium, les polymères quaternaires de vinylpyr-rolidone et de vinylimidazole et leurs mélanges.

Lors d'une étape c), qui peut être mise en oeuvre immédiatement ou non après l'étape b), ou bien après l'étape a), les fibres kératiniques sont traitées avec une composition lavante (B). De préférence, lorsque l'étape c) a lieu après l'étape b), ce traitement a lieu au moins 12 heures après l'application de la composition colorante, de préférence au moins 24 heures après.

La composition lavante (B) comprend à titre de tensioactif, au moins un tensioactif non ionique choisi parmi les alkylpolyglucosides, les tensioactifs monoglycérolés ou polyglycérolés et leurs mélanges.

En ce qui concerne les alkypolyglucosides, ces composés sont bien connus et peuvent être plus particulièrement représenté par la formule générale suivante :

R₁O-(R₂O)ₜ (G)ᵥ

dans laquelle R₁ représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone, R₂ représente un radical alkylène comportant environ de 2 à 4 atomes de carbone, G représente un motif sucre comportant de 5 à 6 atomes de carbone, t désigne une valeur allant de 0 à 10, de préférence 0 à 4, de préférence 0 à 4 et v désigne une valeur allant de 1 à 15.

Des alkylpolyglycosides préférés selon la présente invention sont des composés de formule (II) dans laquelle R₁ désigne plus particulièrement un radical alkyle saturé ou insaturé, linéaire ou ramifié comportant de 8 à 18 atomes de carbone, t désigne une valeur allant de 0 à 3 et plus particulièrement encore égale à 0, G peut désigner le glucose, le fructose ou le galactose, de préférence le glucose. Le degré de polymérisation, i.e. la valeur de v dans la formule (II), peut aller de 1 à 15, de préférence de 1 à 4. Le degré moyen de polymérisation est plus particulièrement compris entre 1 et 2.
Les liaisons glycosidiques entre les motifs sucre sont de type 1-6 ou 1-4 et de préférence 1-4.

Des composés de formule (II) sont notamment représentés par les produits vendus par la société COGNIS sous les dénominations PLANTAREN^{®} (600 CS/U, 1200 et 2000) ou PLANTACARE^{®} (818, 1200 et 2000). On peut également utiliser les produits vendus par la société SEPPIC sous les dénominations TRITON CG 110 (ou ORAMIX CG 110) et TRITON CG 312 (ou ORAMIX^{®} NS 10) , les produits vendus par la société B.A.S.F. sous la dénomination LUTENSOL GD 70 ou encore ceux vendus par la société CHEM Y sous la dénomination AG10 LK.

On peut également utiliser par exemple, l'Alkyl en C8/C16 polyglucoside 1,4 en solution aqueuse à 53% commercialisé par COGNIS sous la référence PLANTACARE^{®} 818 UP. En ce qui concerne les tensioactifs mono ou polyglycérolés, ils comportent de préférence en moyenne de 1 à 30 groupements glycérol, plus particulièrement de 1 à 10 groupements glycérol et en particulier de 1,5 à 5.

Les tensioactifs monoglycérolés ou polyglycérolés sont de préférence choisis parmi les composés de formule suivantes : RO[CH₂CH(CH₂OH)O]ₘH , RO[CH₂CH(OH)CH₂O]ₘH ou RO[CH(CH₂OH)CH₂O]ₘH ; dans laquelle R représente un radical hydrocarboné saturé ou insaturé, linéaire ou ramifié, comportant de 8 à 40 atomes de carbone et de préférence de 10 à 30 atomes de carbone ; m est un nombre compris entre 1 et 30, de préférence entre 1 et 10, plus particulièrement de 1,5 à 6.

R peut éventuellement comprendre des hétéroatomes tels que par exemple oxygène et azote. En particulier, R peut éventuellement comprendre un ou plusieurs groupements hydroxy et/ou éther et/ou amide.

R désigne de préférence des radicaux alkyle et/ou alkényle en C10-C20, éventuellement mono ou polyhydroxylé.

On peut utiliser par exemple, l'hydroxylauryléther polyglycérolé (3.5 moles) commercialisé sous la dénomination Chimexane® NF de Chimex.

Conformément à un mode de réalisation particulier de l'invention, la teneur totale en tensioactif non ionique est comprise entre 4 et 50 % en poids par rapport au poids de la composition lavante (B), de préférence entre 6 et 30 % en poids par rapport au poids de la composition lavante (B).

La composition lavante (B) peut comprendre en outre des tensioactifs additionnels choisis parmi les tensioactifs anioniques, amphotères, zwittérioniques et leurs mélanges.

Ce qui a été indiqué précédemment au sujet de la nature des tensioactifs amphotères, zwittérioniques et anioniques doux reste valable et ne sera pas repris à nouveau.

Les tensioactifs anioniques peuvent être des tensioactifs anioniques doux tels que ceux décrits ci-dessus mais aussi des tensioactifs anioniques forts tels que les alkyléthersulfates, les alkyl sulfates.

Si des tensioactifs additionnels sont présents, alors la teneur totale en tensioactif(s) détergent(s) de ce type est comprise entre 4 et 50% en poids, par rapport à la teneur totale en tensioactifs détergents présents dans la composition lavante (B), plus particulièrement compris entre 6% et 30 % en poids, par rapport à la même référence.

La teneur totale en tensioactif amphotère, zwittérionique, anionique est généralement comprise entre 1 et 20 % en poids par rapport au poids de la composition lavante (B), de préférence entre 1 et 15 % en poids par rapport au poids de la composition lavante (B).

De préférence, la composition lavante (B) ne contient pas de tensioactif détergent anionique fort de type alkylsulfate ou alkylether sulfate. Et si elle en contient, sa teneur est telle que le rapport en poids : tensioactif détergent anionique de type alkylsulfate ou alkylether sulfate / somme des tensioactifsamphotère, zwittérionique, anionique doux et/ou non ionique soit inférieur ou égal à 0,6, et plus particulièrement inférieur ou égal à 0,2.

Selon un mode de réalisation préféré de l'invention, la composition lavante (B) comprend au moins un agent conditionneur, de préférence cationique.
On pourra se reporter à la description de ces composés donnée dans le cadre de la description de la deuxième composition lavante (B).

Selon ce mode de réalisation préféré, la teneur en agent conditionneur dans la deuxième composition lavante est comprise entre 0.01 et 20% en poids par rapport au poids de la première composition lavante.

Selon un autre mode de réalisation préféré de l'invention, la composition lavante (B) comprend au moins un agent épaississant identique à ceux décrits pour la composition lavante (A).

Lorsqu'un agent épaississant est employé, sa teneur dans la deuxième composition lavante (B) est comprise entre 0,01 et 20 % en poids par rapport au poids, de la composition lavante (B).

Enfin, la composition lavante (B) peut comprendre les additifs classiques dans le domaine, et notamment ceux mentionnés dans le cadre de la composition lavante (A), dans les teneurs habituelles indiquées auparavant.

Une fois le ou les lavages réalisés, on sèche les fibres ou on les laisse sécher.

Conformément à une première variante de l'invention, l'étape c) du procédé, c'est-à-dire le lavage mettant en oeuvre la composition lavante (B), est réalisée directement à l'issue de l'étape a) du procédé (étape de coloration).
Ainsi, les fibres kératiniques sont rincées pour éliminer l'excès de composition colorante puis ensuite lavées une ou plusieurs fois avec la composition lavante (B), avec habituellement un rinçage intermédiaire.

A noter que l'étape c) peut être mise en oeuvre plusieurs fois, avec la fréquence classiquement utilisée entre deux shampooings (c'est-à-dire par exemple avec des intervalles de temps compris entre 12 heures et 2 semaines).

Selon une autre possibilité, on met en oeuvre l'étape c) de lavage avec la composition lavante (B), de manière différée par rapport à une étape b) antérieure et/ou par rapport à une étape c) antérieure.

Dans ce cas, le procédé consiste à mettre en oeuvre successivement, les étapes a) et b), ou a) et c), puis à l'issue de la seconde étape (soit l'étape b) soit l'étape c)) à sécher ou à laisser les fibres. L'étape c) est ensuite mise en oeuvre après un temps plus ou moins long, de préférence au moins 12 heures après l'application, ou encore de préférence au moins 24 heures après l'application de la composition colorante.

Un autre objet de la présente invention est constitué par un kit pour la mise en oeuvre du procédé selon l'invention comprenant tout d'abord une composition colorante et éventuellement une composition comprenant au moins un agent oxydant ; puis éventuellement au moins une composition lavante (A) et enfin au moins une composition lavante (B).

Tout ce qui a été précédemment détaillé concernant la nature des éléments constitutifs des diverses compositions ainsi que leurs proportions, reste valable et ne sera pas repris dans cette partie de la description.

Enfin, un autre objet de l'invention est constitué par l'utilisation d'une composition lavante comprenant, à titre de tensioactif, au moins un tensioactif non ionique choisi parmi les alkylpolyglycosides, les tensioactifs monoglycérolés ou polyglycérolés, ou leurs mélanges, pour la protection de la coloration et/ou pour la limitation du dégorgement de la coloration, de fibres kératiniques, coloration obtenue au moyen d'une composition de coloration comprenant au moins un colorant direct cationique.

De préférence, la teneur totale en tensioactifs non ioniques est comprise entre 4 et 50 % en poids par rapport au poids de la composition lavante, de préférence entre 6 et 30 % en poids par rapport au poids de la composition lavante.

En outre, cette composition lavante peut comprendre au moins un tensioactif détergent choisi parmi les tensioactifs amphotères, zwittérioniques, anioniques additionnel de préférence choisi parmi les alkyléthers sulfate et/ou les alkyl sulfate.

De préférence, la composition lavante ne contient pas de tensioactif détergent anionique de type alkylsulfate ou alkylether sulfate. Et si elle en contient, sa teneur est telle que le rapport en poids : tensioactif détergent anionique de type alkylsulfate ou alkylether sulfate / somme des tensioactifs non ioniques soit inférieur ou égal à 0,6, et plus particulièrement inférieur ou égal à 0,2.

Il est noter que la composition lavante peut comprendre au moins un agent conditionneur, de préférence cationique.
Par ailleurs, au cas où la composition comprend un tel agent, sa teneur est comprise entre 0.01 et 20 % en poids par rapport au poids de la composition lavante.

Il est rappelé que l'on pourra se reporter à la description, pour ce qui est de la nature plus précise des ingrédients compris dans cette composition lavante.

Des exemples concrets mais non limitatifs de invention vont maintenant être présentés.

### EXEMPLES

### Etapes de Coloration :

Les compositions de teinture, détaillées dans le tableau 1 suivant, sont réalisées à partir de deux types de colorants directs cationiques :
- colorant cationique dont la charge est portée par un hétérocycle : **Basic Red 51** (Vibracolor Ruby Red - Ciba Geigy)
- colorant cationique ne contenant pas d'hétérocycle : **Basic Red 76** (Arianor Madder Red 306003 - LCW)

Au moment de l'emploi, chacune des compositions ci dessus est mélangée avec de l'eau oxygénée (Eau oxygénée l'Oréal professionnel 20 volumes à 6%) poids par poids.

**Tableau 1**

| | **Coloration 1** | **Coloration 2** |
|---|---|---|
| Décyl glucoside en solution aqueuse à 60% d'Oramix® CG 110 (SEPPIC) | 5,4g | 5,4g |
| Alcool éthylique 96 degrés dénaturé | 18g | 18g |
| Alcool benzylique | 1,8g | 1,8g |
| Polyéthylène glycol (8 OE) | 2,7g | 2,7g |
| Pentasodium pentetate à 40% dans l'eau | 1,08g | 1,08g |
| **Basic Red 51** | 0,25g | - |
| **Basic Red 76** | - | 0,25g |
| Ammoniaque à 20.5% | 13g | 13g |
| Eau déminéralisée | Qsp 100 | Qsp 100 |

Le mélange est ensuite appliqué sur des mèches de cheveux pigmentées moyennement décolorées, à raison de 10 g de mélange colorant/ gramme de mèche. Le temps de pause est de 15 minutes de chaque côté de la mèche.

La coloration est ensuite stoppée par un rinçage à l'eau, suivi d'un lavage avec un shampooing commercial contenant comme tensioactif anionique, le sodium laureth sulfate (shampooing DOP camomille). Puis les mèches sont séchées 30 minutes à 60°C au casque.

### Etapes de lavage

48 heures après la réalisation des colorations précédemment décrites, deux types de shampooings sont évalués :
Les compositions sont détaillées dans le tableau 2 :

**Tableau 2**

| | **shampooing 1** | **shampooing 2** |
|---|---|---|
| Sodium laureth sulfate à 70% dans l'eau Texapon® N 702 (Cognis) | - | 21,43g |
| Coco-glucoside à 53% dans l'eau (Plantacare® 818 UP-Cognis) | 28,30g | - |
| Acide citrique | qsp pH=7 | qsp pH=7 |
| Eau | qsp 100 | qsp 100 |

Les conditions d'applications sont les suivantes :
Après avoir été humidifiée par de l'eau (3 passages entre les doigts sous l'eau), chaque mèche est essorée entre deux doigts et une quantité de 0,4 g de shampooing / gramme de cheveu est appliquée le long de la mèche de cheveux (de la racine à la pointe, de façon homogène).

On fait alors mousser en malaxant doucement la mèche entre deux doigts dans sa longueur pendant 15 secondes de haut en bas (sans faire de noeuds). Puis la mèche est enroulée autour des doigts et disposée dans la coupelle en plastique.

On laisse alors pauser pendant un temps chronométré à 5 minutes pour chaque application et la tenue de la mousse est observée. Le niveau de tenue est chiffré (0 absence de mousse après 5 minutes, 5 bonne tenue de mousse).
Les mèches sont alors rincées, essorées entre deux doigts, peignées et séchées au casque 30 minutes à 60°C.

### Résultats :

Les résultats sont rassemblés dans le tableau 3 suivant :
1. Tenue de la mousse :

Après 5 minutes, une nette amélioration de la tenue de la mousse est notée avec l'alkylpolyglucoside par rapport au sodium laureth sulfate.

D'autre part, on observe qu'avec l'alkylpolyglucoside, la mousse reste nettement plus abondante sur la coloration au Basic Red 51.

**Tableau 3**

| Shampooings appliqués | Shampooing 1 (contenant le Coco-Glucoside) | | Shampooing 2 (contenant le sodium laureth sulfate) | |
|---|---|---|---|---|
| Coloration | Coloration 1 (Basic Red 51) | Coloration 2 (basic Red76) | Coloration 1 (Basic Red 51) | Coloration 2 (Basic Red76) |
| Tenue de mousse t=1 sh-5mn | 5 | 3 | 0 | 0 |

Par ailleurs, on a appliqué chaque shampooing 10 fois et l'on a évalué, après chaque application, d'une part, le dégorgement de la couleur dans l'eau de rinçage et dans la mousse, ainsi que la tenue de la coloration, qui a été observée visuellement 48 heures après séchage des mèches.

On constate que le dégorgement de la couleur dans l'eau et dans la mousse est moins important dans le cas de la présente invention, ce qui se traduit par un moindre tâchage des serviettes, des oreillers, ...

On observe aussi que la tenue de la coloration appliquée selon le procédé de l'invention est plus importante.

## Revendications

1. Procédé de traitement de fibres kératiniques humaines dans lequel on met en oeuvre les étapes suivantes :
a) on réalise une étape de coloration desdites fibres au moyen d'une composition prête à l'emploi à base d'une composition colorante comportant au moins un colorant direct cationique possédant au moins un groupement hétérocyclique ;
b) éventuellement, on lave les fibres avec une composition lavante (A) comprenant au moins un tensioactif détergent anionique choisi parmi les alkyléthers sulfate et/ou les alkyl sulfate, on les rince et éventuellement on les sèche ou on les laisse sécher ;
c) on lave les fibres avec une composition lavante (B) comprenant au moins un tensioactif non ionique choisi parmi les alkylpolyglucosides, les tensioactifs monoglycérolés ou polyglycérolés et leurs mélanges, puis on rince les fibres et on les sèche ou on les laisse sécher.

2. Procédé selon la revendication précédente, **caractérisé en ce que** la composition colorante comprend au moins un colorant direct cationique possédant un hétérocycle portant au moins une charge cationique.

3. Procédé selon la revendication précédente, **caractérisé en ce que** la teneur totale en colorants directs cationiques représente 0,0001 à 20% en poids par rapport au poids de la composition colorante, plus particulièrement de 0,001 à 10% en poids, et de préférence de 0,01 à 5% en poids, par rapport au poids total de la composition colorante.

4. Procédé selon l'une quelconque des revendications précédentes, **caractérisé en ce que** la composition colorante comprend au moins une base d'oxydation et éventuellement au moins un coupleur.

5. Procédé selon la revendication précédente, **caractérisé en ce que** la teneur totale en base d'oxydation est comprise entre 0,0005 et 12% en poids par rapport au poids de la composition colorante, et de préférence de 0,005 à 8% en poids par rapport au poids total de la composition colorante.

6. Procédé selon l'une des revendications 4 ou 5, **caractérisé en ce que** la teneur totale en coupleur représente de 0,0001 à 10% en poids par rapport au poids de la composition colorante, de préférence de 0,005 à 5% en poids par rapport au poids de la composition colorante.

7. Procédé selon l'une quelconque des revendications précédentes, **caractérisé en ce que** la composition prête à l'emploi comprend au moins un agent oxydant.

8. Procédé selon la revendication précédente, **caractérisée en ce que** l'agent oxydant est choisi parmi le peroxyde d'hydrogène, le peroxyde d'urée, les bromates ou ferricyanures de métaux alcalins, les persels, les enzymes d'oxydoréduction telles que les laccases, les peroxydases et les oxydoréductases à 2 électrons, le cas échéant en présence de leur donneur respectif.

9. Procédé selon l'une des revendications précédentes, **caractérisé en ce que** la composition prête à l'emploi est obtenue par mélange avant l'application sur les fibres, d'une composition colorante avec une composition comprenant au moins un agent oxydant.

10. Procédé selon l'une quelconque des revendications précédentes, **caractérisé en ce que** la teneur en tensioactif détergent anionique dans la composition lavante (A) est comprise entre 4 à 50% en poids par rapport au poids de la première composition lavante et de préférence de 6 à 30% en poids par rapport au poids de la composition lavante (A).

11. Procédé selon l'une quelconque des revendications précédentes, **caractérisé en ce que** la composition lavante (B) comprend au moins un alkylpolyglucoside représenté par la formule générale suivante :
R₁O-(R₂O)ₜ(G)ᵥ
dans laquelle R₁ représente un radical alkyle et/ou alcényle linéaire ou ramifié comportant environ de 8 à 24 atomes de carbone, un radical alkylphényle dont le radical alkyle linéaire ou ramifié comporte de 8 à 24 atomes de carbone, R₂ représente un radical alkylène comportant environ de 2 à 4 atomes de carbone, G représente un sucre réduit comportant de 5 à 6 atomes de carbone, t désigne une valeur allant de 0 à 10, de préférence 0 à 4, de préférence 0 à 4, de préférence 0 à 4, et v désigne une valeur allant de 1 à 15.

12. Procédé selon l'une quelconque des revendications précédentes, **caractérisé en ce que** la composition lavante (B) comprend au moins un tensioactif monoglycérolé ou polyglycérolé de formule suivantes : RO[CH₂CH(CH₂OH)O]ₘH, RO[CH₂CH(OH)CH₂O]ₘH ou RO[CH(CH₂OH)CH₂O]ₘH ; dans laquelle R représente un radical hydrocarboné saturé ou insaturé, linéaire ou ramifié, comportant de 8 à 40 atomes de carbone et de préférence de 10 à 30 atomes de carbone ; m est un nombre compris entre 1 et 30, de préférence entre 1 et 10.

13. Procédé selon la revendication 12, **caractérisé en ce que** R désigne de préférence des radicaux alkyle et/ou alkényle en C10-C20, éventuellement mono ou polyhydroxylé.

14. Procédé selon la revendication précédente, **caractérisé en ce que** la teneur totale en tensioactif non ionique est comprise entre 4 et 50 % en poids par rapport au poids de la composition lavante (B), de préférence entre 6 et 30 % en poids par rapport au poids de la composition lavante (B).

15. Procédé selon l'une quelconque des revendications précédentes, **caractérisé en ce que** la composition lavante (B) comprend, en outre au moins un tensioactif choisi parmi les tensioactifs amphotères ou zwittérioniques, les tensioactifs anioniques et leur mélanges.

16. Procédé selon la revendication précédente, **caractérisé en ce que** la composition lavante (B) comprend au moins un tensioactif amphotère ou zwittérionique choisi parmi les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant ; les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes, seuls ou en mélange.

17. Procédé selon l'une quelconque des revendications 14 ou 15, **caractérisé en ce que** la composition lavante (B) comprend au moins un tensioactif anionique choisi parmi, seul ou en mélange :
• les acides alkyl éther carboxyliques polyoxyalkylénés,
• les acides alkylaryl éther carboxyliques polyoxyalkylénés,
• les acides alkylamido éther carboxyliques polyoxyalkylénés en particulier ceux comportant 2 à 50 groupements oxyde d'éthylène,
• les acides d'alkyl D galactoside uroniques
• les acylsarcosinates, les acylglutamates ;
• les esters d'alkylpolyglycosides carboxyliques ;
• les sels d'acides gras.

18. Procédé selon l'une quelconque des revendications 14 à 17, **caractérisé en ce que** la teneur totale en tensioactif amphotère, zwittérionique, anionique est comprise entre 1 et 20 % en poids par rapport au poids de la composition lavante (B), de préférence entre 1 et 15 % en poids par rapport au poids de la composition lavante (B).

19. Procédé selon l'une quelconque des revendications précédentes, **caractérisée en ce que** la composition lavante (A) et/ou la composition lavante (B) comprend au moins un agent conditionneur, de préférence cationique.

20. Procédé selon la revendication précédente, **caractérisé en ce que** l'agent conditionneur est un polymère cationique ou une silicone.

21. Procédé selon la revendication précédente, **caractérisé en ce que** l'agent conditionneur est une silicone aminée.

22. Procédé selon l'une des revendications 19 à 21, **caractérisé en ce que** la teneur en agent conditionneur dans les compositions lavantes (A) et/ou (B) est comprise entre 0,01 et 20 % en poids par rapport au poids, respectivement des compositions lavantes (A) et/ou (B).

23. Procédé selon l'une quelconque des revendications précédentes, **caractérisée en ce que** les compositions lavantes (A) et/ou (B) comprend au moins un agent épaississant.

24. Procédé selon la revendication précédente, **caractérisée en ce que** la teneur en agent épaississant dans les compositions lavantes (A) et/ou (B) est comprise entre 0,01 et 20% en poids par rapport au poids, respectivement des compositions lavantes (A) ou (B).

25. Procédé selon l'une quelconque des revendication précédente, **caractérisé en ce que** la composition lavante (B) est conditionnée dans un dispositif aérosol.

26. Procédé selon la revendication précédente, **caractérisé en ce que** le dispositif contient un propulseur.

27. Procédé selon la revendication précédente, **caractérisé en ce que** le propulseur est choisi parmi les gaz comprimés ou liquéfiés.

28. Procédé selon l'une quelconque des revendications précédentes, **caractérisé en ce que** l'étape c) du procédé est réalisée directement à l'issue de l'étape a) du procédé.

29. Procédé selon la revendication précédente, **caractérisé en ce que** l'on rince les fibres kératiniques pour éliminer l'excès de composition colorante puis on lave lesdites fibres une ou plusieurs fois avec la composition lavante (B), avec habituellement un rinçage intermédiaire.

30. Procédé selon l'une quelconque des revendications précédentes, **caractérisé en ce que** l'application de la composition lavante (B) s'effectue au moins 12 heures après l'application de la composition colorante, de préférence au moins 24 heures après l'application.

31. Procédé selon l'une des revendications précédentes, **caractérisé en ce que** l'étape c) est mise en oeuvre plusieurs fois, avec la fréquence classiquement utilisée entre deux shampooings.

32. Procédé selon la revendication précédente, **caractérisé en ce que** la fréquence est comprise entre 12 heures et 2 semaines.

33. Procédé selon l'une quelconque des revendications 1 à 32, **caractérisé en ce que** l'on met en oeuvre l'étape c), de manière différée par rapport à une étape b) antérieure et/ou par rapport à une étape c) antérieure.

34. Procédé selon la revendication précédente, **caractérisé en ce que** l'on met en oeuvre successivement, les étapes a) et b), ou a) et c), puis à l'issue de la seconde étape, soit l'étape b) soit l'étape c), à sécher ou à laisser les fibres ; puis on met en oeuvre l'étape c).

35. Procédé selon la revendication précédente, **caractérisé en ce que** l'étape c) est mise en oeuvre au moins 12 heures après l'application, ou encore de préférence au moins 24 heures après l'application de la composition colorante.

36. Kit pour la mise en oeuvre du procédé selon l'une quelconque des revendications précédentes, **caractérisé en ce qu'**il comprend une composition colorante comprenant au moins un colorant direct cationique comportant au moins un groupement hétérocyclique et éventuellement une composition comprenant au moins un agent oxydant ; éventuellement au moins une composition lavante (A) comprenant au moins un tensioactif détergent anionique choisi parmi les alkyléthers sulfate et/ou les alkyl sulfate, et au moins une composition lavante (B) comprenant, à titre de tensioactif, au moins un tensioactif non ionique choisi parmi les alkylpolyglycosides, les tensioactifs monoglycérolés ou polyglycérolés, ou leurs mélanges.

37. Utilisation d'une composition lavante comprenant, à titre de tensioactif, au moins un tensioactif non ionique choisi parmi les alkylpolyglycosides, les tensioactifs monoglycérolés ou polyglycérolés, ou leurs mélanges, pour la limitation du dégorgement et/ou l'amélioration de la protection de la coloration de fibres kératiniques, en relation avec l'utilisation préalable d'une composition de coloration comprenant au moins un colorant direct cationique comportant au moins un groupement hétérocyclique.

38. Utilisation selon la revendication précédente, **caractérisée en ce que** la teneur totale en tensioactif non ionique est comprise entre 4 et 50 % en poids par rapport au poids de la composition lavante, de préférence entre 6 et 30 % en poids par rapport au poids de la composition lavante.

39. Utilisation selon l'une quelconque des revendications 37 ou 38, **caractérisée en ce que** la composition lavante comprend au moins un agent conditionneur, de préférence cationique.

40. Utilisation selon la revendications précédente, **caractérisée en ce que** l'agent conditionneur est un polymère cationique et/ou une silicone aminée.

41. Utilisation selon l'une quelconque des revendications 39 ou 40, **caractérisé en ce que** la teneur en agent conditionneur est comprise entre 0,01 et 20% en poids par rapport au poids de la composition lavante.

## Claims

1. Process for treating human keratin fibres, in which the following steps are performed:
a) a step of dyeing the said fibres is performed using a ready-to-use composition based on a dye composition comprising at least one cationic direct dye containing at least one heterocyclic group;
b) optionally, the fibres are washed with a washing composition (A) comprising at least one anionic detergent surfactant chosen from alkyl ether sulfates and/or alkyl sulfates, and the fibres are rinsed and optionally dried or left to dry;
c) the fibres are washed with a washing composition (B) comprising at least one nonionic surfactant chosen from alkylpolyglucosides and monoglycerolated or polyglycerolated surfactants, and mixtures thereof, and the fibres are then rinsed and dried or left to dry.

2. Process according to the preceding claim, **characterized in that** the dye composition comprises at least one cationic direct dye containing a heterocycle bearing at least one cationic charge.

3. Process according to the preceding claim, **characterized in that** the total content of cationic direct dyes represents 0.0001% to 20% by weight relative to the weight of the dye composition, more particularly from 0.001% to 10% by weight and preferably from 0.01% to 5% by weight relative to the total weight of the dye composition.

4. Process according to any one of the preceding claims, **characterized in that** the dye composition comprises at least one oxidation base and optionally at least one coupler.

5. Process according to the preceding claim, **characterized in that** the total content of oxidation base is between 0.0005% and 12% by weight relative to the weight of the dye composition, and preferably from 0.005% to 8% by weight relative to the total weight of the dye composition.

6. Process according to either of Claims 4 and 5, **characterized in that** the total content of coupler represents from 0.0001% to 10% by weight relative to the weight of the dye composition, and preferably from 0.005% to 5% by weight relative to the weight of the dye composition.

7. Process according to any one of the preceding claims, **characterized in that** the ready-to-use composition comprises at least one oxidizing agent.

8. Process according to the preceding claim, **characterized in that** the oxidizing agent is chosen from hydrogen peroxide, urea peroxide, alkali metal bromates or ferricyanides, persalts, redox enzymes such as laccases, peroxidases and 2-electron oxidoreductases, where appropriate in the presence of the respective donor thereof.

9. Process according to one of the preceding claims, **characterized in that** the ready-to-use composition is obtained by mixing, before application to the fibres, a dye composition with a composition comprising at least one oxidizing agent.

10. Process according to any one of the preceding claims, **characterized in that** the content of anionic detergent surfactant in the washing composition (A) is between 4% and 50% by weight relative to the weight of the first washing composition, and preferably from 6% to 30% by weight relative to the weight of the washing composition (A).

11. Process according to any one of the preceding claims, **characterized in that** the washing composition (B) comprises at least one alkylpolyglucoside represented by the following general formula:
R₁O-(R₂O)ₜ(G)ᵥ
in which R₁ represents a linear or branched alkyl and/or alkenyl radical containing from about 8 to 24 carbon atoms, an alkylphenyl radical in which the linear or branched alkyl radical contains from 8 to 24 carbon atoms, R₂ represents an alkylene radical containing from about 2 to 4 carbon atoms, G represents a reduced sugar containing 5 or 6 carbon atoms, t denotes a value ranging from 0 to 10 and preferably from 0 to 4, and v denotes a value ranging from 1 to 15.

12. Process according to any one of the preceding claims, **characterized in that** the washing composition (B) comprises at least one monoglycerolated or polyglycerolated surfactant of the following formulae: RO[CH₂CH(CH₂OH)O]ₘH, RO[CH₂CH(OH)CH₂O]ₘH or RO[CH(CH₂OH)CH₂O]ₘH; in which R represents a saturated or unsaturated, linear or branched hydrocarbon-based radical containing from 8 to 40 carbon atoms and preferably from 10 to 30 carbon atoms; m is an integer between 1 and 30, preferably between 1 and 10.

13. Process according to Claim 12, **characterized in that** R preferably denotes optionally monohydroxylated or polyhydroxylated C₁₀-C₂₀ alkyl and/or alkenyl radicals.

14. Process according to the preceding claim, **characterized in that** the total content of nonionic surfactant is between 4% and 50% by weight relative to the weight of the washing composition (B), and preferably between 6% and 30% by weight relative to the weight of the washing composition (B).

15. Process according to any one of the preceding claims, **characterized in that** the washing composition (B) also comprises at least one surfactant chosen from amphoteric or zwitterionic surfactants and anionic surfactants, and mixtures thereof.

16. Process according to the preceding claim, **characterized in that** the washing composition (B) comprises at least one amphoteric or zwitterionic surfactant chosen from aliphatic secondary or tertiary amine derivatives, in which the aliphatic radical is a linear or branched chain containing from 8 to 18 carbon atoms and at least one water-solubilizing anionic group; (C₈-C₂₀) alkylbetaines, sulfobetaines, (C₈-C₂₀) - alkylamido(C₁-C₆)alkylbetaines and (C₈-C₂₀)alkylamido(C₁-C₆)alkylsulfobetaines, alone or as a mixture.

17. Process according to either of Claims 14 and 15, **characterized in that** the washing composition (B) comprises at least one anionic surfactant chosen, alone or as a mixture, from:
• polyoxyalkylenated alkyl ether carboxylic acids;
• polyoxyalkylenated alkylaryl ether carboxylic acids;
• polyoxyalkylenated alkylamido ether carboxylic acids, in particular those comprising from 2 to 50 ethylene oxide groups;
• alkyl-D-galactosiduronic acids;
• acylsarcosinates and acylglutamates;
• alkylpolyglycoside carboxylic esters;
• fatty acid salts.

18. Process according to any one of Claims 14 to 17, **characterized in that** the total content of amphoteric, zwitterionic and anionic surfactants is between 1% and 20% by weight relative to the weight of the washing composition (B), and preferably between 1% and 15% by weight relative to the weight of the washing composition (B).

19. Process according to any one of the preceding claims, **characterized in that** the washing composition (A) and/or the washing composition (B) comprises at least one conditioning agent, which is preferably cationic.

20. Process according to the preceding claim, **characterized in that** the conditioning agent is a cationic polymer or a silicone.

21. Process according to the preceding claim, **characterized in that** the conditioning agent is an amino silicone.

22. Process according to one of Claims 19 to 21, **characterized in that** the content of conditioning agent in the washing composition(s) (A) and/or (B) is between 0.01% and 20% by weight relative to the weight, respectively, of the washing composition(s) (A) and/or (B).

23. Process according to any one of the preceding claims, **characterized in that** the washing composition(s) (A) and/or (B) comprise(s) at least one thickener.

24. Process according to the preceding claim, **characterized in that** the content of thickener in the washing composition(s) (A) and/or (B) is between 0.01% and 20% by weight relative to the weight of the washing composition (A) or (B), respectively.

25. Process according to any one of the preceding claims, **characterized in that** the washing composition (B) is packaged in an aerosol device.

26. Process according to the preceding claim, **characterized in that** the device contains a propellant.

27. Process according to the preceding claim, **characterized in that** the propellant is chosen from compressed or liquefied gases.

28. Process according to any one of the preceding claims, **characterized in that** step c) of the process is performed directly after step a) of the process.

29. Process according to the preceding claim, **characterized in that** the keratin fibres are rinsed to remove the excess dye composition, and the said fibres are then washed one or more times with the washing composition (B), usually with intermediate rinsing.

30. Process according to any one of the preceding claims, **characterized in that** the washing composition (B) is applied at least 12 hours and preferably at least 24 hours after the application of the dye composition.

31. Process according to one of the preceding claims, **characterized in that** step c) is performed several times, at the frequency conventionally used between two shampoo washes.

32. Process according to the preceding claim, **characterized in that** the frequency is between 12 hours and 2 weeks.

33. Process according to any one of Claims 1 to 32, **characterized in that** step c) is performed in a delayed manner relative to a prior step b) and/or relative to a prior step c).

34. Process according to the preceding claim, **characterized in that** steps a) and b) or a) and c) are performed successively and then, after the second step (either step b) or step c)), in drying the fibres or leaving them to dry; step c) is then performed.

35. Process according to the preceding claim, **characterized in that** step c) is performed at least 12 hours after the application, or alternatively preferably at least 24 hours after the application of the dye composition.

36. Kit for performing the process according to any one of the preceding claims, **characterized in that** it comprises a dye composition comprising at least one cationic direct dye comprising at least one heterocyclic group and optionally a composition comprising at least one oxidizing agent, optionally at least one washing composition (A) comprising at least one anionic detergent surfactant chosen from alkyl ether sulfates and/or alkyl sulfates, and at least one washing composition (B) comprising, as surfactant, at least one nonionic surfactant chosen from alkylpolyglycosides and monoglycerolated or polyglycerolated surfactants, or mixtures thereof.

37. Use of a washing composition comprising, as surfactant, at least one nonionic surfactant chosen from alkylpolyglycosides and monoglycerolated or polyglycerolated surfactants, or mixtures thereof, for limiting the bleeding of the coloration and/or for improving the protection of the coloration of keratin fibres, in relation with the prior use of a dye composition comprising at least one cationic direct dye comprising at least one heterocyclic group.

38. Use according to the preceding claim, **characterized in that** the total content of nonionic surfactant is between 4% and 50% by weight relative to the weight of the washing composition, and preferably between 6% and 30% by weight relative to the weight of the washing composition.

39. Use according to either of Claims 37 and 38, **characterized in that** the washing composition comprises at least one conditioning agent, which is preferably cationic.

40. Use according to the preceding claim, **characterized in that** the conditioning agent is a cationic polymer and/or an amino silicone.

41. Use according to either of Claims 39 and 40, **characterized in that** the content of conditioning agent is between 0.01% and 20% by weight relative to the weight of the washing composition.

## Patentansprüche

1. Verfahren zur Behandlung von menschlichen Keratinfasern, bei dem die folgenden Schritte durchgeführt werden:
a) es wird ein Schritt durchgeführt, bei dem die Fasern mit Hilfe einer gebrauchsfertigen Zusammensetzung auf der Basis einer Farbmittelzusammensetzung gefärbt werden, die mindestens einen kationischen Direktfarbstoff enthält, der zumindest eine heterocyclische Gruppe aufweist;
b) wahlweise werden die Fasern mit einer reinigenden Zusammensetzung (A) gewaschen, die mindestens einen anionischen reinigenden grenzflächenaktiven Stoff enthält, der unter den Alkylethersulfaten und/oder Alkylsulfaten ausgewählt ist, gespült und gegebenenfalls getrocknet oder trocknen gelassen;
c) die Fasern werden mit einer reinigenden Zusammensetzung (B) gewaschen, die mindestens einen nichtionischen reinigenden grenzflächenaktiven Stoff enthält, der unter den Alkylpolyglucosiden, ein- oder mehrfach mit Glycerin veretherten grenzflächenaktiven Stoffen und deren Gemischen ausgewählt ist, worauf die Fasern gespült und getrocknet oder trocknen gelassen werden.

2. Verfahren nach dem vorhergehenden Anspruch, **dadurch gekennzeichnet, dass** die Farbmittelzusammensetzung mindestens einen kationischen Direktfarbstoff enthält, der einen Heterocyclus umfasst, der mindestens eine kationische Ladung trägt.

3. Verfahren nach dem vorhergehenden Anspruch, **dadurch gekennzeichnet, dass** die Gesamtmenge an kationischen Direktfarbstoffen im Bereich von 0,0001 bis 20 Gew.-%, bezogen auf das Gesamtgewicht der Farbmittelzusammensetzung, insbesondere im Bereich von 0,001 bis 10 Gew.-% und vorzugsweise im Bereich von 0,01 bis 5 Gew.-%, bezogen auf das Gesamtgewicht der Farbmittelzusammensetzung, liegt.

4. Verfahren nach einem der vorhergehenden Ansprüche, **dadurch gekennzeichnet, dass** die Farbmittelzusammensetzung mindestens eine Oxidationsbase und gegebenenfalls mindestens einen Kuppler enthält.

5. Verfahren nach dem vorhergehenden Anspruch, **dadurch gekennzeichnet, dass** die Gesamtmenge an Oxidationsbase im Bereich von 0,0005 bis 12 Gew.-%, bezogen auf das Gesamtgewicht der Farbmittelzusammensetzung, vorzugsweise im Bereich von 0,005 bis 8 Gew.-%, bezogen auf das Gesamtgewicht der Farbmittelzusammensetzung, liegt.

6. Verfahren nach einem der Ansprüche 4 oder 5, **dadurch gekennzeichnet, dass** die Gesamtmenge an Kuppler 0,0001 bis 10 Gew.-%, bezogen auf das Gesamtgewicht der Farbmittelzusammensetzung, und vorzugsweise 0,005 bis 5 Gew.-%, bezogen auf das Gesamtgewicht der Farbmittelzusammensetzung, ausmacht.

7. Verfahren nach einem der vorhergehenden Ansprüche, **dadurch gekennzeichnet, dass** die gebrauchsfertige Farbmittelzusammensetzung mindestens ein Oxidationsmittel enthält.

8. Verfahren nach dem vorhergehenden Anspruch, **dadurch gekennzeichnet, dass** das Oxidationsmittel unter Wasserstoffperoxid, Harnstoffperoxid, Alkalimetallbromaten oder Alkalimetallferricyaniden, Salzen von Persäuren und Redoxenzymen, wie Laccasen, Peroxidasen und Oxidoreduktasen (2 Elektronen) gegebenenfalls zusammen mit ihrem jeweiligen Donor ausgewählt ist.

9. Verfahren nach einem der vorhergehenden Ansprüche, **dadurch gekennzeichnet, dass** die gebrauchsfertige Zusammensetzung vor dem Auftragen auf die Keratinfasern durch Mischen einer Farbmittelzusammensetzung mit einer Zusammensetzung, die mindestens ein Oxidationsmittel enthält, gebildet wird.

10. Verfahren nach einem der vorhergehenden Ansprüche, **dadurch gekennzeichnet, dass** der Mengenanteil an anionischem reinigenden grenzflächenaktiven Stoff in der reinigenden Zusammensetzung (A) im Bereich von 4 bis 50 Gew.-%, bezogen auf das Gesamtgewicht der ersten reinigenden Zusammensetzung, und vorzugsweise im Bereich von 6 bis 30 Gew.-%, bezogen auf das Gesamtgewicht der reinigenden Zusammensetzung (A), liegt.

11. Verfahren nach einem der vorhergehenden Ansprüche, **dadurch gekennzeichnet, dass** die reinigende Zusammensetzung (B) mindestens ein Alkylpolyglucosid enthält, das durch die folgende allgemeine Formel dargestellt wird:
R₁O-(R₂O)ₜ(G)ᵥ
worin R₁ eine geradkettige oder verzweigte Alkyl- und/oder Alkenylgruppe, die etwa 8 bis 24 Kohlenstoffatome aufweist, oder eine Alkylphenylgruppe bedeutet, deren geradkettige oder verzweigte Alkylgruppe 8 bis 24 Kohlenstoffatome aufweist, R₂ eine Alkylengruppe bedeutet, die etwa 2 bis 4 Kohlenstoffatome aufweist, G ein reduzierter Zucker mit 5 bis 6 Kohlenstoffatome ist, t einen Wert im Bereich von 0 bis 10 und vorzugsweise 0 bis 4 bedeutet und v ein Wert im Bereich von 1 bis 15 ist.

12. Verfahren nach einem der vorhergehenden Ansprüche, **dadurch gekennzeichnet, dass** die reinigende Zusammensetzung (B) mindestens einen ein- oder mehrfach mit Glycerin veretherten grenzflächenaktive Stoff der folgenden Formeln enthält: RO[CH₂CH(CH₂OH)O]ₘH, RO[CH₂CH(OH)CH₂O]ₘH oder RO[CH(CH₂OH)CH₂O]ₘH; wobei R eine gesättigte oder ungesättigte, lineare oder verzweigte Kohlenwasserstoffgruppe mit 8 bis 40 Kohlenstoffatomen und vorzugsweise 10 bis 30 Kohlenstoffatomen bedeutet; und m eine Zahl im Bereich von 1 bis 30 und vorzugsweise 1 bis 10 ist.

13. Verfahren nach Anspruch 12, **dadurch gekennzeichnet, dass** R vorzugsweise Alkyl- und/oder Alkenylgruppen mit 10 bis 10 Kohlenstoffatomen bedeutet, die gegebenenfalls ein- oder mehrfach hydroxyliert sind.

14. Verfahren nach dem vorhergehenden Anspruch, **dadurch gekennzeichnet, dass** die Gesamtmenge an nichtionischem reinigenden grenzflächenaktiven Stoff im Bereich von 4 bis 50 Gew.-%, bezogen auf das Gewicht der reinigenden Zusammensetzung (B), und vorzugsweise im Bereich von 6 bis 30 Gew.-%, bezogen auf das Gewicht der reinigenden Zusammensetzung (B), liegt.

15. Verfahren nach einem der vorhergehenden Ansprüche, **dadurch gekennzeichnet, dass** die reinigende Zusammensetzung (B) ferner mindestens einen grenzflächenaktiven Stoff enthält, der unter den amphoteren oder zwitterionischen grenzflächenaktiven Stoffen, anionischen grenzflächenaktiven Stoffen und deren Gemischen ausgewählt ist.

16. Verfahren nach dem vorhergehenden Anspruch, **dadurch gekennzeichnet, dass** die reinigende Zusammensetzung (B) ferner mindestens einen amphoteren oder zwitterionischen grenzflächenaktiven Stoff enthält, der einzeln oder in Form von Gemischen unter den sekundären oder tertiären, aliphatischen Aminderivaten, bei denen die aliphatische Gruppe eine lineare oder verzweigte Kette ist, die 8 bis 18 Kohlenstoffatome enthält und mindestens eine Wasserlöslichkeit vermittelnde anionische Gruppe aufweist; Alkyl(C₈₋₂₀)betainen, Sulfobetainen, Alkyl(C₈₋₂₀)amidoalkyl(C₁₋₆)betainen oder Alkyl(C₈₋₂₀)amidoalkyl(C₁₋₆)-sulfobetainen ausgewählt ist.

17. Verfahren nach einem der Ansprüche 14 oder 15, **dadurch gekennzeichnet, dass** die reinigende Zusammensetzung (B) mindestens einen anionischen grenzflächenaktiven Stoff enthält, der einzeln oder in Form von Gemischen ausgewählt ist unter:
· polyalkoxylierten Alkylethercarbonsäuren,
· polyalkoxylierten Alkylarylethercarbonsäuren,
· polyalkoxylierten Alkylamidoethercarbonsäuren und insbesondere solchen mit 2 bis 50 Ethylenoxidgruppen,
· Alkyl-D-galactosiduronsäuren,
· Acylsarcosinaten, Acylglutamaten,
· Carbonsäureestern der Alkylpolyglycoside,
· Fettsäuresalzen.

18. Verfahren nach einem der Ansprüche 14 bis 17, **dadurch gekennzeichnet, dass** die Gesamtmenge an amphoterem, zwitterionischen, anionischen grenzflächenaktiven Stoff im Bereich von 1 bis 20 Gew.-%, bezogen auf das Gewicht der reinigenden Zusammensetzung (B), und vorzugsweise im Bereich von 1 bis 15 Gew.-%, bezogen auf das Gewicht der reinigenden Zusammensetzung (B), liegt.

19. Verfahren nach einem der vorhergehenden Ansprüche, **dadurch gekennzeichnet, dass** die reinigende Zusammensetzung (A) und/oder die reinigende Zusammensetzung (B) mindestens ein Konditioniermittel enthalten, das vorzugsweise kationisch ist.

20. Verfahren nach dem vorhergehenden Anspruch, **dadurch gekennzeichnet, dass** es sich bei dem Konditioniermittel um ein kationisches Polymer oder ein Silicon handelt.

21. Verfahren nach dem vorhergehenden Anspruch, **dadurch gekennzeichnet, dass** das Konditioniermittel ein aminiertes Silicon ist.

22. Verfahren nach einem der Ansprüche 19 bis 21, **dadurch gekennzeichnet, dass** der Mengenanteil an Konditioniermittel in den reinigenden Zusammensetzungen (A) und/oder (B) im Bereich von 0,01 bis 20 Gew.-%, bezogen auf das Gewicht der reinigenden Zusammensetzungen (A) und/ oder (B), liegt.

23. Verfahren nach einem der vorhergehenden Ansprüche, **dadurch gekennzeichnet, dass** die reinigenden Zusammensetzungen (A) und/ oder (B) mindestens ein Verdickungsmittel enthalten.

24. Verfahren nach dem vorhergehenden Anspruch, **dadurch gekennzeichnet, dass** der Mengenanteil an Verdickungsmittel in den reinigenden Zusammensetzungen (A) und/oder (B) im Bereich von 0,01 bis 20 Gew.-%, bezogen auf das Gewicht der reinigenden Zusammensetzungen (A) und/oder (B), liegt.

25. Verfahren nach einem der vorhergehenden Ansprüche, **dadurch gekennzeichnet, dass** die reinigende Zusammensetzung (B) in einer Aerosolvorrichtung konfektioniert ist.

26. Verfahren nach dem vorhergehenden Anspruch, **dadurch gekennzeichnet, dass** die Vorrichtung ein Treibmittel enthält.

27. Verfahren nach dem vorhergehenden Anspruch, **dadurch gekennzeichnet, dass** das Treibmittel unter Druckgasen oder Flüssiggasen ausgewählt ist.

28. Verfahren nach einem der vorhergehenden Ansprüche, **dadurch gekennzeichnet, dass** der Schritt c) des Verfahrens direkt nach dem Schritt a) des Verfahrens durchgeführt wird.

29. Verfahren nach dem vorhergehenden Anspruch, **dadurch gekennzeichnet, dass** die Keratinfasern gespült werden, um überschüssige Farbmittelzusammensetzung zu entfernen, worauf die Fasern einmal oder mehrmals mit der reinigenden Zusammensetzung (B) gewaschen werden, wobei gewöhnlich zwischendurch gespült wird.

30. Verfahren nach einem der vorhergehenden Ansprüche, **dadurch gekennzeichnet, dass** die Anwendung der reinigenden Zusammensetzung (B) mindestens 12 Stunden nach der Anwendung der Farbmittelzusammensetzung und vorzugsweise mindestens 24 Stunden nach dieser Anwendung erfolgt.

31. Verfahren nach einem der vorhergehenden Ansprüche, **dadurch gekennzeichnet, dass** Schritt c) mehrmals mit einer Häufigkeit wiederholt wird, wie sie gewöhnlich dem Abstand zwischen zwei Haarwäschen entspricht.

32. Verfahren nach dem vorhergehenden Anspruch, **dadurch gekennzeichnet, dass** die Häufigkeit der Wiederholung im Bereich von 12 Stunden bis 2 Wochen liegt.

33. Verfahren nach einem der Ansprüche 1 bis 32, **dadurch gekennzeichnet, dass** Schritt c) in Bezug auf einen vorhergehenden Schritt b) und/oder in Bezug auf einen vorhergehenden Schritt c) zeitlich verzögert durchgeführt wird.

34. Verfahren nach dem vorhergehenden Anspruch, **dadurch gekennzeichnet, dass** nacheinander die Schritte a) und b) oder die Schritte a) und c) und dann am Ende des zweiten Schritts entweder Schritt b) oder Schritt c) durchgeführt wird, die Fasern getrocknet oder trocknen gelassen werden; und dann Schritt c) durchgeführt wird.

35. Verfahren nach dem vorhergehenden Anspruch, **dadurch gekennzeichnet, dass** Schritt c) mindestens 12 Stunden nach der Anwendung der Farbmittelzusammensetzung und noch bevorzugter mindestens 24 Stunden nach dieser Anwendung durchgeführt wird.

36. Kit für die Durchführung des Verfahrens nach einem der vorhergehenden Ansprüche, **dadurch gekennzeichnet, dass** es eine Farbmittelzusammensetzung, die mindestens einen kationischen Direktfarbstoff enthält, der mindestens eine heterocyclische Gruppe aufweist, und gegebenenfalls eine Zusammensetzung, die mindestens ein Oxidationsmittel enthält, gegebenenfalls mindestens eine reinigende Zusammensetzung (A), die mindestens einen anionischen reinigenden grenzflächenaktiven Stoff enthält, der unter den Alkylethersulfaten und/ oder Alkylsulfaten ausgewählt ist, und mindestens eine reinigende Zusammensetzung (B) enthält, die als grenzflächenaktiven Stoff mindestens einen nichtionischen grenzflächenaktiven Stoff enthält, der unter den Alkylpolyglucosiden, ein- oder mehrfach mit Glycerin veretherten grenzflächenaktive Stoffen und deren Gemischen ausgewählt ist.

37. Verwendung einer reinigenden Zusammensetzung, die als grenzflächenaktiven Stoff mindestens einen nichtionischen grenzflächenaktiven Stoff enthält, der unter den Alkylpolyglucosiden, ein- oder mehrfach mit Glycerin veretherten grenzflächenaktive Stoffen und deren Gemischen ausgewählt ist, um hinsichtlich der vorherigen Verwendung einer Farbmittelzusammensetzung, die mindestens einen kationischen Direktfarbstoff enthält, der mindestens eine heterocyclische Gruppe aufweist, das Ausschwitzen einzuschränken und/oder die Farbe der Fasern besser zu schützen.

38. Verwendung nach dem vorhergehenden Anspruch, **dadurch gekennzeichnet, dass** der Mengenanteil an nichtionischem grenzflächenaktiven Stoff im Bereich von 4 bis 50 Gew.-%, bezogen auf das Gewicht der reinigenden Zusammensetzung, und vorzugsweise im Bereich von 6 bis 30 Gew.-%, bezogen auf das Gewicht der reinigenden Zusammensetzung, liegt.

39. Verwendung nach einem der Ansprüche 37 oder 38, **dadurch gekennzeichnet, dass** die reinigende Zusammensetzung mindestens ein Konditioniermittel enthält, das vorzugsweise kationisch ist.

40. Verwendung nach dem vorhergehenden Anspruch, **dadurch gekennzeichnet, dass** es sich bei dem Konditioniermittel um ein kationisches Polymer und/oder ein aminiertes Silicon handelt.

41. Verwendung nach einem der Ansprüche 39 oder 40, **dadurch gekennzeichnet, dass** der Mengenanteil an Konditioniermittel im Bereich von 0,01 bis 20 Gew.-%, bezogen auf das Gewicht der reinigenden Zusammensetzung, liegt.
